# EUROPEAN PATENT SPECIFICATION

(11) **EP 4 089 033 B1**
(45) Date of publication and mention of the grant of the patent: **18.06.2025**
(21) Application number: 21780197.6
(22) Date of filing: 12.02.2021
(51) Int. Cl.: B65D 85/07, A61F 13/15, A61F 13/42, A61F 13/51, A61F 13/551, A61F 13/84, B65D 30/08, B65D 33/04, B65D 33/25, B65D 75/00

(54) **ACCOMMODATION BODY FOR ABSORBENT ARTICLE**
AUFNAHMEKÖRPER FÜR SAUGFÄHIGEN ARTIKEL
CORPS DE RÉCEPTION POUR ARTICLE ABSORBANT

(30) Priority: 31.03.2020 JP 2020065055; 31.03.2020 JP 2020065056; 31.03.2020 JP 2020065057
(43) Date of publication of application: 16.11.2022
(73) Proprietor: Unicharm Corporation, Ehime 799-0111 (JP)
(72) Inventor: SHIMIZU, Noriko, Kanonji-shi, Kagawa 769-1602 (JP); MIYAMA, Takuya, Kanonji-shi, Kagawa 769-1602 (JP); SOU, Tatsuya, Kanonji-shi, Kagawa 769-1602 (JP)
(74) Representative: Dolleymores
(86) International application number: PCT/JP2021/005215
(87) International publication number: WO 2021/199712

(56) References cited:
- WO-A1-2017/064895
- WO-A1-2018/163867
- WO-A1-2019/003983
- WO-A1-2019/176961
- JP-A- 2000 190 986
- JP-A- 2004 115 118
- JP-A- 2005 008 164
- JP-A- 2005 096 779
- JP-A- 2011 006 142
- JP-A- 2018 102 419
- JP-A- 2019 112 103
- JP-A- 2019 156 465
- KR-A- 20170 026 035
- US-A1- 2006 264 858
- US-A1- 2015 175 312
- US-A1- 2017 042 744

## Description

### FIELD

The present invention relates to an absorbent-article container.

### BACKGROUND

There is known an absorbent-article container including: an containing member having an outermost surface which is at least partially formed of paper; and an absorbent article that is contained in the containing member and includes an absorbent core, and whose outermost surface is at least partially formed of nonwoven fabric (e.g., see Patent Literature 1).

### [CITATION LIST]

### [PATENT LITERATURE]

Patent Literature 1: Japanese Patent Application Publication No. 2019-59552
KR20170026035 discloses individually packaged absorbent articles, each absorbent article contained in an individual wrapper. An outer packinging material contains a plurality of individually packaged absorbent articles.

### SUMMARY

### [TECHNICAL PROBLEM]

A problem to be solved by the present disclosure is at least one of first to third problems below. The first problem is that, since the containing member formed of paper has a higher friction coefficient compared with the containing member formed of a resin sheet or the like, the absorbent article is likely to be caught and fluffed when taking out the absorbent article from the containing member.

The present invention was achieved in light of conventional problems such as that described above, and an aspect of the present invention is to provide a container having an appearance of paper and in which fluffing of an absorbent article at the time of taking out is suppressed.

The second problem is that, if the absorbent article is contained in the containing member formed of a non-transparent material (e.g., paper), when a consumer purchases the absorbent article, the absorbent article cannot be visually recognized from the outside of the container that is displayed or the like.

However, when the container is configured to be capable of visually recognize the absorbent article from the outside (e.g., providing a transparent window), the absorbent article is exposed to ultraviolet light of sunlight which is incident from a portion where the absorbent article can be visually recognized at the time of display or the like. In the case of the absorbent article which has an indicator for detecting excrement, it causes a problem that the indicator or the like deteriorates due to ultraviolet light of sunlight.

An aspect of the present disclosure is to make it possible to visually recognize an absorbent article located inside from outside, and to suppress the deterioration of an indicator by suppressing a reaction of the indicator with ultraviolet light at the time of display or the like.

The third problem is that, if a graphic of product information or the like is printed on the containing member, there is a risk, for example, that when a plurality of containers are packed in one cardboard box or the like, it bring into close contact and strongly compress the containers, particularly, a portion of the absorbent core having a maximum thickness of the absorbent article, and this causes a risk that the graphic is rubbed or ink of the graphic transfers to the absorbent article.

An aspect of the present disclosure is to suppress the rubbing of a graphic between adjacent containers and color transfer of a graphic to an absorbent article, when being packed, in which a plurality of containers are arranged.

A main aspect of the present invention for solving the first problem is an absorbent-article container including: an containing member having an outermost surface, at least a part of the outermost surface being formed of paper; and an absorbent article that is contained in the containing member and that has an outermost surface, at least a part of the outermost surface being formed of nonwoven fabric, a minimum value of dynamic friction coefficient on an innermost surface of the containing member is less than dynamic friction coefficient on the outermost surface of the containing member formed of the paper.

Features of the present invention other than the above will become clear by reading the description of the present specification with reference to the accompanying drawings.

### [ADVANTAGEOUS EFFECT OF THE INVENTION]

An effect of the present invention on the first problem is enabling to provide a container having an appearance of paper and in which fluffing of an absorbent article at the time of taking out is suppressed.

### BRIEF DESCRIPTION OF DRAWINGS

FIG. 1 is a schematic front view and a schematic cross-sectional view of a container 1.
FIG. 2 is a schematic front view and a schematic back view of a containing member 10.
FIG. 3 is a schematic cross-sectional view of the containing member 10.
FIG. 4 is a schematic front view and a schematic back view of a diaper 20.
FIG.5 is a schematic plan view and a schematic cross-sectional view of the diaper 20.
FIG. 6 is a diagram showing a state where the container 1 is reversed in a vertical direction.
FIG. 7 is a diagram illustrating friction coefficient.
FIG. 8 is a diagram showing measurement results of the friction coefficient.
FIG. 9 is a diagram illustrating surface roughness SMD.
FIG. 10 is a diagram showing measurement results of the surface roughness SMD.
FIG. 11 is a diagram illustrating a state in which the diaper 20 is being taken out from the containing member 10.
FIG. 12 is a diagram illustrating how the diaper 20 is taken out with putting a hand into a gap of the containing member 10.
FIG. 13 is a table showing values obtained by measuring the Bekk smoothness of sheet members (samples A to C) of a paper material used as an outermost layer.
FIG. 14A is a diagram showing an arrangement of a graphic 15 provided on the containing member 10.
FIG. 14B is a schematic cross-sectional view showing a cross section taken along a line X-X of FIG. 14A.
FIG. 15 is a diagram illustrating a containing member 110 that includes gusseted portions G1 and G2.

At least following matters will become clear with description of this specification and attached drawings.

An absorbent-article container including: an containing member having an outermost surface, at least a part of the outermost surface being formed of paper; and an absorbent article that is contained in the containing member and that has an outermost surface, at least a part of the outermost surface being formed of nonwoven fabric, a minimum value of dynamic friction coefficient on an innermost surface of the containing member is less than dynamic friction coefficient on the outermost surface of the containing member formed of the paper.

According to the absorbent-article container described above, compared with the case where the dynamic friction coefficient on the innermost surface is larger than the dynamic friction coefficient on the outermost surface, it is possible to suppress the fluffing of the nonwoven fabric at the time of taking out the absorbent article while maintaining the appearance of the container as paper.

In such an absorbent-article container, it is desirable that at least a part of the outermost surface of the absorbent article is a facing surface that faces the containing member, and that the minimum value of dynamic friction coefficient on the innermost surface of the containing member is less than a minimum value of dynamic friction coefficient on the facing surface.

According to the absorbent-article container described above, compared with the case where the dynamic friction coefficient on the innermost surface is larger than the dynamic friction coefficient on the facing surface of the absorbent article, it is possible to suppress the fluffing of the nonwoven fabric at the time of taking out the absorbent article.

In such an absorbent-article container, it is desirable that a dynamic friction coefficient on an outermost surface of the paper is less than a maximum value of dynamic friction coefficient on the outermost surface of the absorbent article.

According to the absorbent-article container described above, compared with the case where the dynamic friction coefficient on the outermost surface is larger than the dynamic friction coefficient on the facing surface of the absorbent article, it is possible to suppress the fluffing of the nonwoven fabric due to contact of the containing member with the absorbent article after the absorbent article is taken out.

In such an absorbent-article container, the absorbent-article container wherein at least a part of the outermost surface of the absorbent article is a facing surface that faces the containing member, and wherein a minimum value of dynamic friction coefficient on a located-farthest-on-skin-side surface of the absorbent article is less than a minimum value of dynamic friction coefficient on the facing surface.

According to the absorbent-article container described above, compared with the case where the dynamic friction coefficient the farthest on the skin side of the absorbent article is larger than the dynamic friction coefficient on the facing surface, a wearer is less likely to feel discomfort when the absorbent article is rubbed while being put on. In addition, when the innermost surface of the containing member and the facing surface of the absorbent article rub, portions of the located-farthest-on-skin-side surface are likely to slide with each other, and accordingly, the facing surface easily moves together with the innermost surface and the fluffing can be suppressed.

In such an absorbent-article container, it is desirable that at least a part of the outermost surface of the absorbent article is a facing surface that faces the containing member, and that a minimum value of dynamic friction coefficient on the facing surface is less than a minimum value of dynamic friction coefficient on a located-farthest-on-skin-side surface of the absorbent article.

According to the absorbent-article container described above, compared with the case where the dynamic friction coefficient on the facing surface of the absorbent article is larger than the dynamic friction coefficient on the located-farthest-on-skin-side surface, the absorbent article is easily taken out from the containing member.

In such an absorbent-article container, it is desirable that a minimum value of surface roughness on the innermost surface of the containing member is less than surface roughness on the outermost surface of the containing member formed of the paper.

According to the absorbent-article container described above, in the innermost surface of the containing member, the surface roughness is small, and this suppresses damages on the absorbent article due to rubbing. In the outermost surface, the surface roughness is large, and this makes the outermost surface more likely to be caught by fingers, making it easier to hold the container.

In such an absorbent-article container, it is desirable that at least a part of the outermost surface of the absorbent article is a facing surface that faces the containing member, and that a minimum value of surface roughness on the innermost surface of the containing member is less than a minimum value of surface roughness on the facing surface.

According to the absorbent-article container described above, in the innermost surface of the containing member, the surface roughness is small, and this suppresses damages on the absorbent article due to rubbing. In the facing surface of the absorbent article, the surface roughness is large, and this makes the facing surface more likely to be caught by fingers, making it easier to take out the absorbent article from the containing member.

In such an absorbent-article container, it is desirable that the absorbent-article container has a vertical direction, a lateral direction, and a front-back direction that intersect with each other, that an upper end portion of the containing member is provided with an openable portion that is opened when taking out the absorbent article, and that in a state where the container is upright with a lower end of the container being in contact with a ground plane, a gap is provided between the absorbent article and the openable portion.

According to the absorbent-article container described above, it is possible to put a hand into the gap, and this makes it easier to spread the containing member with fingers, making the absorbent article less likely to be rubbed when taking out.

In such an absorbent-article container, it is desirable that the openable portion is positioned below an upper end of the containing member.

According to the absorbent-article container described above, compared with the case where the openable portion is provided on the upper end of the containing member, a further gap is formed (the gap + a portion from the openable portion to the upper end), and this makes it easier to further spread the containing member with fingers, making the absorbent article further less likely to be rubbed when taking out.

In such an absorbent-article container, it is desirable that the container contains a plurality of the absorbent articles, and that the absorbent articles are arranged side by side in the front-back direction, but not being arranged side by side in the vertical direction and the lateral direction.

According to the absorbent-article container described above, compared with the case where the absorbent articles are arranged in two or more rows, there is no case where one row and another row come into contact with each other, which makes it possible to suppress the collapse of the alignment of the rows.

In such an absorbent-article container, it is desirable that the containing member has a two-layer structure including the paper and a resin sheet, and that the innermost surface of the containing member is formed of the resin sheet.

According to the absorbent-article container described above, the innermost surface is a smooth surface, which makes it possible to suppress the fluffing of the nonwoven fabric when the absorbent article is taken out from the containing member of the absorbent article.

In such an absorbent-article container, it is desirable that the absorbent-article container has a vertical direction, a lateral direction, and a front-back direction that intersect with each other, that the containing member includes a non-window region and a window region, the non-window region being a region in which the paper and the resin sheet overlap, the window region being a region having only the resin sheet, and that the window region is provided continuously along the lateral direction from a lateral one end to a lateral other end.

According to the absorbent-article container described above, the window region having small stiffness is provided continuously from the lateral one end to the lateral other end, and therefore a change in stiffness at a boundary between the window region and the non-window region occurs through from the lateral one end to the lateral other end. This makes such a portion more likely to be fold in the vertical direction, and the folding makes the absorbent article less likely to move in the containing member, making it possible to suppress the fluffing.

In such an absorbent-article container, it is desirable that in a state where the container is upright with a lower end of the container being in contact with a ground plane, an upper end and a lower end of the window region overlap the absorbent article when seen in the front-back direction.

According to the absorbent-article container described above, the boundary between the window region and the non-window region is likely to be folded due to a change in stiffness. Accordingly, making the upper end and the lower end overlap the absorbent article makes such a portion less likely to be folded.

In such an absorbent-article container, it is desirable that in a state where the container is upright with a lower end of the container being in contact with a ground plane, an upper end of the window region is positioned above a vertical center of the absorbent article that is in an contained state.

According to the absorbent-article container described above, a boundary between the window region and the non-window region is more likely to be folded due to a change in stiffness. This easily makes the container compact when disposing, by putting the used absorbent article into the container with the used absorbent article being folded one time in the vertical direction, for example.

In such an absorbent-article container, it is desirable that the absorbent-article container has a vertical direction, a lateral direction, and a front-back direction that intersect with each other, that the containing member includes a front side portion, a back side portion, and a bottom surface portion, that a gusset for forming the bottom surface portion is provided between a lower end portion of the front side portion and a lower end portion of the back side portion, and that the gusset is not provided in an upper end portion of the containing member.

According to the absorbent-article container described above, since the lower end portion cannot be open, the lower side of the containing member is spread by providing the gusset in advance, which makes it possible to suppress the rubbing of the containing member and the absorbent article when taking out the absorbent article.

In such an absorbent-article container, it is desirable that the absorbent article includes a front portion that comes into contact with a stomach side of a wearer, and a back portion that comes into contact with a back side of the wearer, that the front portion and the back portion face each other, and that the absorbent article is contained in the containing member so that a crotch side of the absorbent article is positioned on a lower side of the container.

According to the absorbent-article container described above, the lower side of the containing member is provided with a bottom surface sheet and is wide. Accordingly, placing the absorbent article (diaper) so that the crotch side, which is thick, is located in the lower side, makes it easier to enter the absorbent article.

In such an absorbent-article container, it is desirable that the absorbent article includes a front portion that comes into contact with a stomach side of a wearer, and a back portion that comes into contact with a back side of the wearer, that the front portion and the back portion face each other, and that the absorbent article is contained in the containing member so that a crotch side of the absorbent article is positioned on an upper side of the container.

According to the absorbent-article container described above, since the crotch side of the absorbent article (diaper) has high stiffness, placing the absorbent article in the container so that the crotch side is located in the upper side makes it easier to pull the absorbent article when taking out.

An absorbent-article container having a vertical direction, a lateral direction, and a front-back direction that intersect with each other, the absorbent-article container including: an containing member; and an absorbent article that is contained in the containing member and that includes an indicator for detecting excrement, the containing member including a window region through which the absorbent article is capable of being visually recognized from outside of the containing member, and a non-window region through which the absorbent article is not capable of being visually recognized, in a state where the container is upright with a lower end of the container being in contact with a ground plane, at least a part of the indicator overlaps the non-window region when seen in the front-back direction.

According to the absorbent-article container described above, it is possible to visually recognize the absorbent article located inside from outside. Further, since the indicator overlap the non-window region at the time of display or the like, this suppresses a reaction of the indicator with ultraviolet light, making it possible to suppress the deterioration of the indicator.

In such an absorbent-article container, it is desirable that a product graphic is printed on the absorbent article, and that in the state where the container is upright with the lower end of the container being in contact with a ground plane, at least a part of the product graphic is capable of being visually recognized from the outside of the containing member through the window region.

According to the absorbent-article container described above, it enables the consumer to check the product graphic of the absorbent article without opening it at the time of display or the like.

In such an absorbent-article container, it is desirable that the product graphic includes a mark indicating at least any one of followings: a name of country in which the absorbent article has been manufactured; a logo representing a name of a company that manufactures or sells the absorbent article; a logo representing a product name of the absorbent article; and a material used in the absorbent article.

According to the absorbent-article container described above, without opening it at the time of display or the like, it enables the consumer to check at least one of the followings: the country in which the absorbent article has been manufactured; the logo of the name of the manufacturing company; the logo of the product name; and the material used.

In such an absorbent-article container, it is desirable that the absorbent article includes: a front portion that comes into contact with a stomach side of a wearer; and a back portion that comes into contact with a back side of the wearer, and that, in the state where the container is upright with the lower end of the container being in contact with a ground plane, either one of the front portion and the back portion faces the window region.

According to the absorbent-article container described above, the consumer can check the front surface or the back surface having a larger area than the side surface of the absorbent article (diaper), without opening it at the time of display or the like.

In such an absorbent-article container, it is desirable that the absorbent article includes a front portion that comes into contact with a stomach side of a wearer, and a back portion that comes into contact with a back side of the wearer, that the front portion and the back portion face each other, that the absorbent article is contained so that a crotch side of the absorbent article is positioned on a lower side of the container, that the absorbent article has a crotch region, the crotch region being a region located lowest when the absorbent article in an contained state is divided into four equal regions in the vertical direction, and that in the state where the container is upright with the lower end of the container being in contact with a ground plane, at least a part of the crotch region overlaps the non-window region when seen in the front-back direction.

According to the absorbent-article container described above, since the indicator is particularly required in the crotch region, making the crotch region and the non-window region overlap suppresses the reaction of the indicator with ultraviolet light at the time of display or the like. This makes it possible to suppress the deterioration of the indicator.

In such an absorbent-article container, it is desirable that the indicator of the absorbent article includes a portion that is positioned on the front portion and a portion that is positioned on the back portion, that either one of the front portion and the back portion faces the window region, and that the portion of the indicator positioned on the other one of the front portion and the back portion extends upward in the vertical direction longer than the portion of the indicator positioned on the either one of the front portion and the back portion.

According to the absorbent-article container described above, compared with the case where a portion of the indicator having a long length faces the window region, it suppresses the reaction of the indicator with ultraviolet light at the time of display or the like (a portion of the indicator that overlaps the window region is reduced). This makes it possible to suppress the deterioration of the indicator.

In such an absorbent-article container, it is desirable that the indicator of the absorbent article includes a portion that is positioned on the front portion and a portion that is positioned on the back portion, that either one of the front portion and the back portion faces the window region, and that the portion of the indicator positioned on the either one of the front portion and the back portion extends upward in the vertical direction longer than the portion of the indicator positioned on the other one of the front portion and the back portion.

According to the absorbent-article container described above, compared with the case where a portion of the indicator having a short length faces the window region, it makes the consumer easier to visually recognize the indicator from outside at the time of display or the like, while suppressing the deterioration of the crotch region in the non-window region.

In such an absorbent-article container, it is desirable that in the state where the container is upright with the lower end of the container being in contact with a ground plane, a vertical length of an overlapping portion where the non-window region overlaps the portion of the indicator positioned on the either one of the front portion and the back portion is longer than a vertical length of an overlapping portion where the window region overlaps the portion of the indicator positioned on the either one of the front portion and the back portion.

According to the absorbent-article container described above, the portion of the indicator that has a long length is capable of being visually recognized from the window region, and simultaneously, making longer the length of the region overlapping with the non-window region can suppress the deterioration of the indicator, compared with the case of being short.

In such an absorbent-article container, it is desirable that in the state where the container is upright with the lower end of the container being in contact with a ground plane, a vertical length of an overlapping portion where the indicator and the non-window region overlap is longer than a vertical length of an overlapping portion where the indicator and the window region overlap.

According to the absorbent-article container described above, compared with the case where the length overlapping with the window region is longer, the reaction of the indicator and with ultraviolet light is suppressed at the time of display, which makes it possible to suppress the deterioration of the indicator.

In such an absorbent-article container, it is desirable that the containing member has a portion having a two-layer structure that is composed of paper and a resin sheet.

According to the absorbent-article container described above, the paper can suppress the deterioration of the indicator caused by ultraviolet light, and the resin sheet can suppress the deterioration of the absorbent core caused by moisture or the like.

In such an absorbent-article container, it is desirable that the window region is a region composed of only the resin sheet on which a layer of the paper is not overlaid, that an upper end of the window region is positioned below an upper end of the containing member, that a lower end of the window region is positioned above a lower end of the containing member, and that the window region is provided continuously along the lateral direction from a lateral one end to a lateral other end.

According to the absorbent-article container described above, the window region is provided up to an end portion in the width direction, and accordingly, the consumer easily visually recognizes the absorbent article located inside.

In such an absorbent-article container, it is desirable that in the state where the container is upright with the lower end of the container being in contact with a ground plane, the upper end and the lower end of the window region overlap the absorbent article when seen in the front-back direction.

According to the absorbent-article container described above, the boundary between the window region and the non-window region is likely to be folded due to a change in stiffness. Accordingly, making the upper end and the lower end overlap the absorbent article makes the boundary less likely to be folded.

In such an absorbent-article container, it is desirable that in the state where the container is upright with the lower end of the container being in contact with a ground plane, the upper end of the window region is positioned above a vertical center of the absorbent article that is in an contained state.

According to the absorbent-article container described above, a boundary between the window region and the non-window region is more likely to be folded due to a change in stiffness. This easily makes the container compact when disposing, by putting the used absorbent article into the container with the used absorbent article being folded one time in the vertical direction, for example.

In such an absorbent-article container, it is desirable that in the state where the container is upright with the lower end of the container being in contact with a ground plane, the upper end of the window region does not overlap the absorbent article when seen in the front-back direction.

According to the absorbent-article container described above, it enables the consumer to check the absorbent article up to the one end thereof without opening it at the time of display or the like.

In such an absorbent-article container, it is desirable that in a state where a vertical relationship is reversed with respect to the state where the container is upright with the lower end of the container being in contact with a ground plane, at least a part of the indicator overlaps the non-window region when seen in the front-back direction.

According to the absorbent-article container described above, in a state where the container is reversed (since the containing member is formed of paper, there is a gap inside; that is, in a state where the absorbent article moves upward inside the container), the reaction of the indicator with the ultraviolet light is suppressed, and this makes it possible to suppress the deterioration of the indicator.

In such an absorbent-article container, it is desirable that the paper is unbleached.

According to the absorbent-article container described above, unbleached paper has a larger content of lignin than bleached paper. Therefore, compared with the case where the bleached paper is used, the reaction between the indicator and ultraviolet light is suppressed, and this makes it possible to suppress the deterioration of the indicator.

In such an absorbent-article container, it is desirable that the containing member includes a front side portion, a back side portion, and a bottom surface portion, that a gusset for forming the bottom surface portion is provided between a lower end portion of the front side portion and a lower end portion of the back side portion, that the gusset is not provided on an upper end portion of the containing member, and that in a lower end portion of the containing member, the bottom surface portion is joined to at least one of the front side portion and the back side portion.

According to the absorbent-article container described above, the number of layers where members join in the lower end portion of the containing member is greater than the number of layers where members join in the upper end portion of the containing member. Therefore, the lower end portion of the containing member is reinforced, making it possible to suppress the exposure of the indicator due to the damage on the lower end portion and to suppress the deterioration of the indicator.

In such an absorbent-article container, it is desirable that the absorbent article includes a front portion that comes into contact with a stomach side of a wearer, and a back portion that comes into contact with a back side of the wearer, the front portion and the back portion face each other, and that the absorbent article is contained in the containing member so that a crotch side of the absorbent article is positioned on a lower side of the container.

According to the absorbent-article container described above, since the crotch side of the diaper 20 is thick, a portion of the containing member 10 where the crotch side is positioned becomes likely to tear. But, compared with the case where the crotch side is placed on the upper side of the containing member 10, the number of layers where members join in the lower end portion of the containing member 10 is greater than the number of layers where members join in the upper end portion (the lower end portion is more securely joined). This makes it possible to suppress the exposure of the indicator 23 due to the damage on the containing member 10 and to suppress the deterioration of the indicator 23.

An absorbent-article container including: an containing member; and an absorbent article that is contained in the containing member and that includes an absorbent core, a graphic is printed on the containing member, and in a state where the absorbent article is positioned at a certain position in the containing member, at least a part of the graphic does not overlap the absorbent core when seen in a thickness direction of the absorbent article.

According to the absorbent-article container described above, when being packed, in which a plurality of containers are arranged, it is possible to suppress the rubbing of the graphic between adjacent containers and the color transfer of the graphic to the absorbent article.

In such an absorbent-article container, it is desirable that in a state where the absorbent article is positioned at a certain position in the containing member, at least a part of the graphic does not overlap the absorbent article when seen in the thickness direction of the absorbent article.

According to the absorbent-article container described above, when being packed, in which a plurality of containers are arranged, it is possible to suppress the rubbing of the graphic between adjacent containers and the color transfer of the graphic to the absorbent article.

In such an absorbent-article container, it is desirable that in a state where the absorbent article is positioned at a certain position in the containing member, a region where the graphic and the absorbent article do not overlap when seen in the thickness direction of the absorbent article is larger than a region where the graphic and the absorbent article overlap when seen in the thickness direction of the absorbent article.

According to the absorbent-article container described above, compared with the case where the non-overlapping region is small, it is possible to suppress the rubbing of the graphic or the color transfer of the graphic to the absorbent article.

In such an absorbent-article container, it is desirable that at least a part of an outermost surface of the containing member is formed of paper, that the containing member has an internal space inside, that the internal space has a space where the absorbent article is present and a gap space where the absorbent article is not present, and that in a state where the absorbent article is positioned at a certain position in the containing member, at least a part of the graphic overlaps the gap space when seen in the thickness direction of the absorbent article.

According to the absorbent-article container described above, since the absorbent article is not present in the gap space, it is possible to suppress the rubbing of the graphic that overlaps such a portion or the color transfer of the graphic to the absorbent article.

In such an absorbent-article container, it is desirable that the containing member has a two-layer structure including the paper and a resin sheet, and that a layer of the resin sheet is provided inside a layer of the paper.

According to the absorbent-article container described above, the resin sheet makes it possible to suppress the infiltration of ink into the internal space, which makes it possible to suppress the color transfer of graphic to the absorbent article.

In such an absorbent-article container, it is desirable that a minimum value of static friction coefficient on an innermost surface of the containing member is less than static friction coefficient on the outermost surface of the containing member formed of the paper.

According to the absorbent-article container described above, compared with the case where the minimum value of the static friction coefficient on the innermost surface is large, the absorbent article in the internal space is likely to move inside due to shaking of the container during transport or the like. This makes it possible to suppress the movement of the containing member accompanying with the movement of the absorbent article, which makes it possible to suppress the rubbing of the graphic of the containing member.

In such an absorbent-article container, it is desirable that the absorbent-article container has a vertical direction, a lateral direction, and a front-back direction that intersect with each other, that the containing member includes a front side portion, a back side portion, and a bottom surface portion, that a gusset for forming the bottom surface portion is provided between a lower end portion of the front side portion and a lower end portion of the back side portion, that the gusset is not provided on an upper end portion of the containing member, and that at least a part of the graphic is positioned on an upper portion of the containing member.

According to the absorbent-article container described above, the distance between the front side portion and the back side portion at the lateral center is shorter in the upper portion than the lower portion. Accordingly, printing the graphic on the upper portion makes it possible to widen the space between the graphics of adjacent containers, which makes it possible to suppress the rubbing of the graphic of the containing member or the color transfer of the graphic to the absorbent article.

In such an absorbent-article container, it is desirable that the graphic is printed on only either one of the front side portion and the back side portion.

According to the absorbent-article container described above, compared with the case where the printing is made on both surfaces, it is possible to suppress the rubbing of the graphic of the containing member or the color transfer of the graphic to the absorbent article.

In such an absorbent-article container, it is desirable that the absorbent-article container has a vertical direction, a lateral direction, and a front-back direction that intersect with each other, that an upper end portion of the containing member is provided with an openable portion that is opened when taking out the absorbent article, and that the graphic is not printed on the openable portion.

According to the absorbent-article container described above, since the graphic is not printed on the openable portion, which a hand comes into contact with when opening the absorbent article, it is possible to suppress the transfer of ink to the hand and the being faint of the graphic.

In such an absorbent-article container, it is desirable that the openable portion is capable of resealing after being opened.

According to the absorbent-article container described above, the absorbent article can be put into and taken out by resealing many times. Therefore, by not printing the graphic on the openable portion that comes into contact many times when putting it in and taking it out, it is possible to further suppress the transfer of ink to the hand and the being faint of the graphic.

In such an absorbent-article container, it is desirable that the openable portion is positioned below an upper end of the containing member, and that the graphic is not printed on an upper side of the openable portion.

According to the absorbent-article container described above, the absorbent article can be put into and taken out by resealing many times. Therefore, by not printing the graphic on the upper side of the openable portion that comes into contact many times when putting it in and taking it out, it is possible to further suppress the transfer of ink to the hand and the being faint of the graphic.

In such an absorbent-article container, it is desirable that the graphic is a containing-portion graphic, that a product graphic is printed on the absorbent article, and that a maximum ink density of the containing-portion graphic is higher than a maximum ink density of the product graphic.

According to the absorbent-article container described above, compared with the case where the ink density of the graphic is low, the graphic can be securely displayed, and making it easier to visually recognize the graphic (when the ink density is high, the color transfer or the like is more likely to occur, but by making the graphic and the absorbent core not overlap each other, or the like, it is possible to suppress the rubbing of the graphic of the containing member or the color transfer of the graphic to the absorbent article). In addition, compared with the case where the density of the product graphic is high, this makes the consumer to be more likely to have skin-friendly visual impression on the absorbent article.

### Embodiment

In the present embodiment, the following describes the absorbent-article container by way of example a container 1 (hereinafter, also simply referred to as the container 1) that contains the diaper 20 for newborn infant (hereinafter, also simply referred to as the diaper 20). It should be noted that the absorbent article of the present embodiment includes, for example, a diaper for adult, a sanitary napkin, and the like, and is not limited to the diaper 20.

### Container 1 and containing member 10

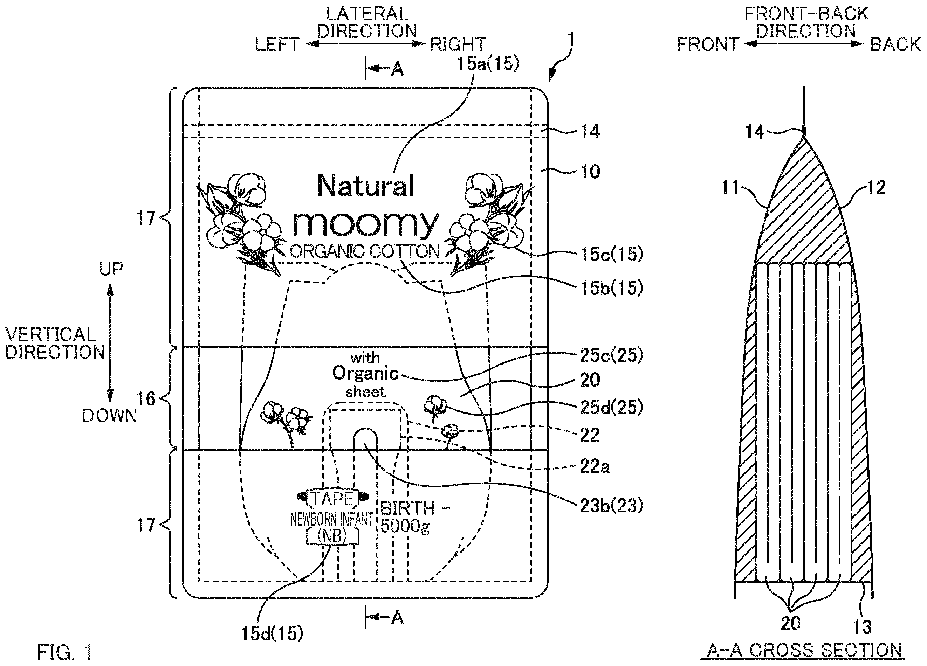

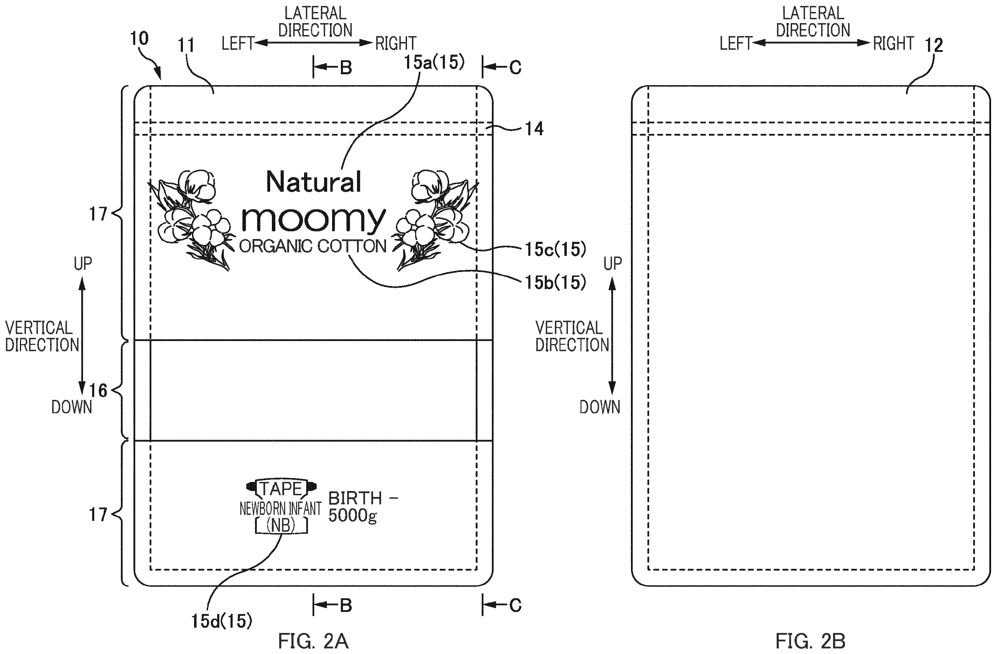

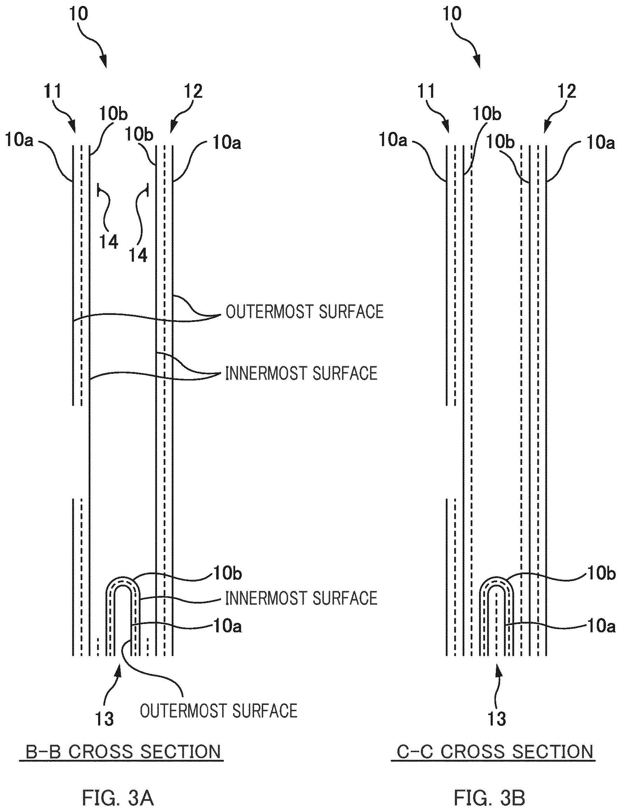

FIG. 1 is a schematic front view of the container 1 and a schematic cross-sectional view taken along a line A-A. FIG. 2 is a schematic front view and a schematic back view of a containing member 10, in which FIG. 2A is the schematic front view, and FIG. 2B is the schematic back view. FIG. 3 is a schematic cross-sectional view of the containing member 10, in which FIG. 3A is a schematic cross-sectional view taken along a line B-B in FIG. 2A, and FIG. 3B is a schematic cross-sectional view taken along a line C-C in FIG. 2A. In addition, portions indicated by dashed lines in FIG. 3 are joining portions in which members are joined.

In the drawings, the container 1 has a vertical direction, a front-back direction, and a lateral direction that intersect with each other. In addition, regarding the vertical direction, in a state where letters and the like of a graphic 15 of the containing member 10 can be correctly recognized and mascot illustrations and the like naturally appear, the upper side of the container 1 is defined as "up" and the opposite side thereof is defined as "down". In addition, FIG. 1 shows a state where the container 1 is upright with the lower end of the container 1 being in contact with a ground plane.

The container 1 includes: the containing member 10 having an outermost surface (see FIG. 3A), at least a part of the outermost surface being formed of paper 10a; and diapers 20 that are contained in the containing member 10 and each of which has an outermost surface, at least a part of the outermost surface being formed of nonwoven fabric (later-described exterior body 21). The diaper 20 includes: an indicator 23 for detecting excrement; and an absorbent core 22a.

The outermost surface of the containing member 10 is formed of the paper 10a, which has a small stretchability, and therefore a gap space (region indicated by upward-right hatched portions in the schematic cross-sectional view of FIG. 1) is formed inside of the containing member 10 (internal space). That is, at least a part of the outermost surface of the containing member 10 is formed of the paper 10a, the containing member 10 has the internal space therein, and the internal space has a space where the diaper 20 is present and a gap space where the diaper 20 is not present.

The internal space of the containing member 10 is formed by shaping the containing member 10 into a three-face shape. That is, the containing member 10 includes a front side portion 11, a back side portion 12, and a bottom surface portion 13. Between the lower end portion of the front side portion 11 and the lower end portion of the back side portion 12, a gusset for forming the bottom surface portion 13 is provided, and the gusset is not provided in an upper end portion of the containing member 10.

In addition, in the containing member 10, in two lateral end portions, the front side portion 11 and the back side portion 12 are joined (welded) along the vertical direction, and in the lower end portion, the front side portion 11, the back side portion 12, and the bottom surface portion 13 are joined (welded) along the lateral direction. That is, in the lower end portion of the containing member 10, the bottom surface portion 13 is joined to at least one of the front side portion 11 and the back side portion 12.

In addition, on the upper end portion of the containing member 10, an opening-and-closing seal portion 14 (corresponding to an openable portion) that is opened when the diaper 20 is taken out is provided. In addition, the opening-and-closing seal portion 14 can be resealed after opening, and can be opened and closed a plurality of times. It should be noted that the opening-and-closing seal portion 14 may be provided on an upper end of the containing member 10, but the opening-and-closing seal portion 14 according to the present embodiment is positioned below the upper end of the containing member 10.

In addition, in a state shown in FIG. 1, a gap (gap space) is generated between the opening-and-closing seal portion 14 and the diaper 20. That is, in a state where the container 1 is upright with the lower end of the container 1 being in contact with a ground plane, the gap is provided between the diaper 20 and the opening-and-closing seal portion 14.

In addition, as shown in FIG. 1, four diapers 20 are contained in the internal space of the containing member 10, and the diapers 20 are arranged side by side in a thickness direction of the diaper 20. That is, the container 1 contains a plurality of diapers 20, and the diapers 20 are arranged side by side in the front-back direction, but are not arranged side by side in the vertical direction and the lateral direction.

In addition, as shown in FIG. 3, the containing member 10 has a two-layer structure including the paper 10a and a resin sheet 10b (has a two-layer structure portion composed of the paper 10a and the resin sheet 10b), and a layer of the resin sheet 10b is provided inside a layer of the paper 10a. That is, the innermost surface of the containing member 10 is formed of the resin sheet 10b. It should be noted that, as described above, the dashed line shown in FIG. 3 indicates a joining portion. The joining between the paper 10a and the resin sheet 10b can form a joining portion by laminating, for example. The joining between the members in the front side portion 11, the back side portion 12, and the bottom surface portion 13 (the resin sheet 10b and the resin sheet 10b) can form (weld) a joining portion by thermal fusion, for example.

As the paper 10a that constitutes the containing member 10, it is possible to use one defined in "JIS P 0001:1998 Paper, board and pulp-Vocabulary, No. 4004, Vocabulary: paper". In addition, as the paper 10a according to the present embodiment, an unbleached paper (brown paper that has not been subjected to treatment such as bleaching) is used.

It should be noted that, although the containing member 10 is formed of a composite material in which a material other than the paper 10a is used, it is desirable that, in the mass of the containing member 10, the proportion of the mass of the paper 10a that constitutes the outermost layer exceeds 50%. By doing so, even when the containing member 10 is formed of the composite material, the proportion of the mass of the paper 10a exceeds 50%, which makes the paper 10a a main material of the containing member 10. The containing member 10 can be treated as subject to a reference mark that can be disposed as paper waste (not illustrated, so-called "paper" mark).

As the resin in the resin sheet 10b that constitutes the containing member 10, it is possible to use a natural resin or a synthetic resin. As the natural resin, it is possible to use a resin derived from plants, animals, or minerals, and for example pine butter, lacquer, or the like are included. As the synthetic resin, it is possible to use a high-molecular-weight resin-like substance obtained by polycondensation or the like from a low-molecular-weight compound having a fossil resource as a raw material, and for example a thermoplastic resin or a thermosetting resin are included. As the thermoplastic resin, it is possible to use polyethylene, polypropylene, polyvinyl chloride, polystyrene, polycarbonate, polysulfone, polyamide, polyphenylene oxide, or the like. As the thermosetting resin, it is possible to use a urea resin, a melamine resin, a phenolic resin, an epoxy resin, or the like.

In addition, the resin includes biomass plastic. The "biomass plastic" is a plastic obtained by synthesizing a renewable biomass resource, which is used as a raw material. Generally, biomass plastics have a so-called carbon-neutral property that it does not increase the concentration of CO₂ in the air even in a case of being incinerated at the time of disposal, and accordingly are environmentally-friendly materials. Accordingly, the inclusion of biomass plastic in the resin that constitutes the innermost layer makes it possible to decrease the proportion of petroleum-derived substances in the innermost layer by an amount thereof. Therefore, in the present embodiment, it is desirable that the weight proportion of the biomass plastic included in the resin that constitutes the innermost layer is as large as possible. Specifically, the weight proportion of the biomass plastic is desirably 10% or more, and the weight proportion is more desirably 25% or more.

In addition, the resin sheet 10b that constitutes the containing member 10 includes a film having a thickness smaller than that of the sheet. That is, generally, a thickness of 200 µm or less is sometimes referred to as a film, and the resin sheet 10b according to the present embodiment includes such a film. It should be noted that it is preferable that the resin sheet 10b has moisture permeability.

In addition, as shown in FIG. 2A, the graphic 15 is printed on the front side portion 11 (front surface side) of the containing member 10. It should be noted that the graphic 15 is provided in order to make a consumer visually recognize information and the like relating to the container 1 (diaper 20), and is printed on an upper portion and a lower portion of the containing member 10. That is, at least a part of the graphic 15 is positioned on the upper portion of the containing member 10. It should be noted that, it is preferable that the graphic 15 of the containing member 10 is printed more widely on the upper portion (upper half) than on the lower portion (lower half).

In the present embodiment, the following graphics are provided as the graphic 15: a first graphic 15a displaying a name of the diaper 20 (product name: Natural moomy); a second graphic 15b displaying a feature portion (material used: organic cotton) of the diaper 20; a third graphic 15c displaying a wearer suitable for wearing the diaper 20 (newborn infant, birth to 5,000 g); and a fourth graphic 15d drawing a flower pattern.

The graphic 15 is printed on the front surface side of the outermost layer by gravure printing or flexographic printing with a predetermined aqueous ink. Generally, for the aqueous ink, water is used as a solvent in many cases, and thus the aqueous ink is an environmentally friendly ink, since it is safe and has little odor compared with an oily ink using an organic solvent or the like. Meanwhile, the aqueous ink has a weak fixing property to a printed medium compared with the oily ink, and therefore, in some cases, it has been difficult to use the aqueous ink for a product to be distributed to the market. In contrast, in the present embodiment, since the outermost layer which is a printed medium is formed of the paper 10a, it is easy to fix the aqueous ink, making it possible to stably form the graphic 15. Therefore, also from a viewpoint of environmental consideration, it is suitable to use the aqueous ink as the ink for printing the graphic 15 on the containing member 10 of the present embodiment.

In addition, as shown in FIGS. 1 to 3, the containing member 10 is provided with a window region 16 through which the diaper 20 can be visually recognized from the outside and a non-window region 17 through which the diaper 20 cannot be visually recognized. In addition, the window region 16 is a region composed of only the resin sheet 10b, on which the layer of the paper 10a is not overlaid, and the non-window region 17 is a region where the paper 10a and the resin sheet 10b are overlaid on each other. That is, the containing member 10 has the non-window region 17 where the paper 10a and the resin sheet 10b overlap, and the window region 16 of only the resin sheet 10b.

In addition, the window region 16 is continuously provided substantially horizontally from the left end to the right end in the lateral direction, and is provided below the upper end and above the lower end of the containing member 10. That is, the upper end of the window region 16 is positioned below the upper end of the containing member 10, the lower end of the window region 16 is positioned above the lower end of the containing member 10, and the window region 16 is provided continuously along the lateral direction from the lateral one end to the lateral other end.

### Diaper 20

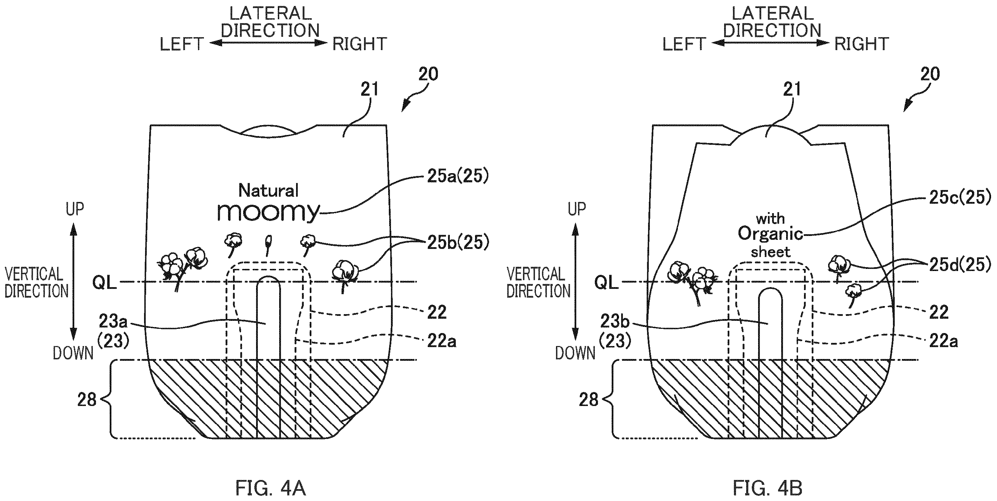

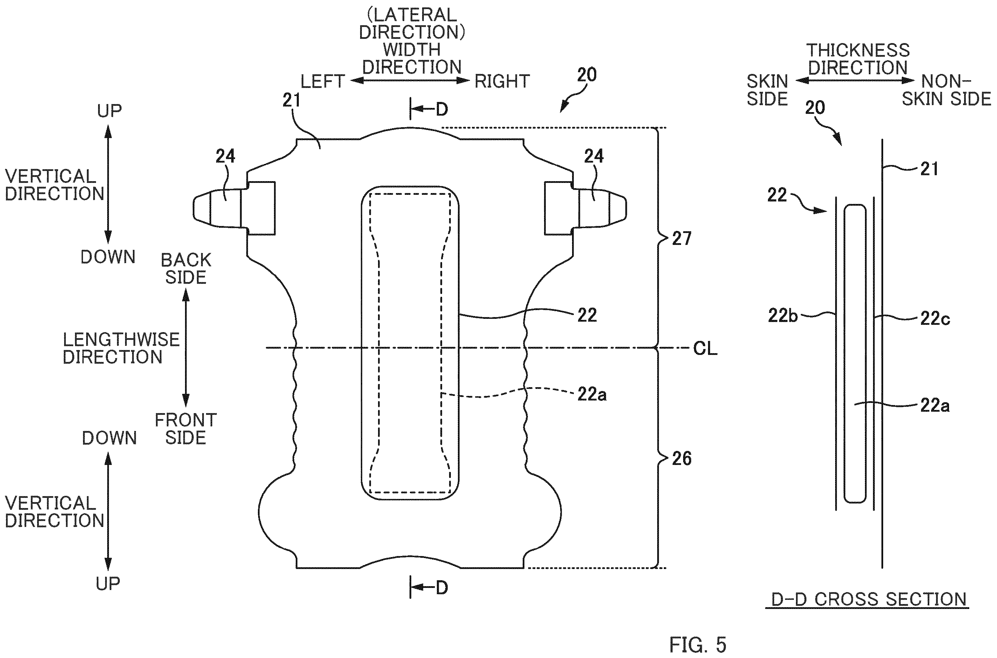

FIG. 4 is a schematic front view and a schematic back view of the diaper 20, in which FIG. 4A is the schematic front view, and FIG. 4B is the schematic back view. FIG.5 is a schematic plan view and a schematic cross-sectional view of the diaper 20. In a state of FIG. 4, the diaper 20 has a vertical direction and a lateral direction that are orthogonal to each other. In addition, hereinafter, the upper side and the lower side in the vertical direction will also be referred to as a "waist side" and a "crotch side", respectively.

In addition, in an unfolded state of FIG. 5, the diaper 20 has three directions that are orthogonal to each other, namely a lengthwise direction, a width direction, and a thickness direction. Further, Hereinafter, the one side and the other side in the lengthwise direction in the unfolded state will also be referred to as the "front side" and the "back side", respectively. It should be noted that the width direction in the unfolded state is a direction extending along the lateral direction of the container 1, and the lengthwise direction is a direction extending along the vertical direction. In addition, as shown in FIG. 5, in the thickness direction, the side that comes into contact with skin of a target wearer is referred to as the "skin side", and the opposite side thereof is referred to as the "non-skin side".

The diaper 20 includes: the exterior body 21; an absorbent main body 22 having a rectangular shape in a plan view and positioned on the skin side of the exterior body 21; an indicator 23; and a tape 24.

In addition, as shown in FIG. 5, the diaper 20 includes a front portion 26 and a back portion 27. The front portion 26 is a portion positioned on the wearer's front side when the diaper 20 is put on, and is positioned on the front side with respect to the central position CL in the lengthwise direction in FIG. 5. The back portion 27 is a portion positioned on the wearer's back side when the diaper 20 is put on, and is positioned on the back side with respect to the central position CL in the lengthwise direction in FIG. 5.

In addition, in the contained state of the diaper 20, the front portion 26 and the back portion 27 of the diaper 20 are folded so as to face each other, and the diaper 20 is contained with the crotch side being located on the lower side of the container 1. That is, the diaper 20 includes the front portion 26 that comes into contact with the wearer's front side and the back portion 27 that comes into contact with the wearer's back side, the front portion 26 and the back portion 27 face each other, and the diaper 20 is contained in the containing member 10 so that the crotch side of the diaper 20 is positioned on the lower side of the container 1. In addition, in the present embodiment, the diaper 20 is contained so that the back portion 27 of the diaper 20 faces the window region 16.

It should be noted that, in the diaper 20 of the present embodiment, the front portion 26 and the back portion 27 are formed as a single unit as shown in FIG. 5, but the front portion 26 and the back portion 27 may be separate members. For example, in the vicinity of the central position CL between the front portion and the back portion in the lengthwise direction, a crotch portion (not shown) positioned in the wearer's crotch portion may be provided, and the diaper may be composed of the front portion, the back portion, and the crotch portion.

For example, the diaper 20 can be put on a wearer as follow: bringing the back portion 27 into contact with the wearer's back side (buttocks or the like), and bringing the front portion 26 into contact with the wearer's stomach side (lower abdomen or the like) through the space between both legs (through under the crotch), and then joining an adhesive surface of the tape 24 provided on the back portion 27 to the exterior body 21 of the front portion 26.

The exterior body 21 is a portion that constitutes the exterior of the diaper 20, and is formed by overlaying a plurality of flexible sheet-like members in the thickness direction. In the present embodiment, a nonwoven fabric sheet is used as a flexible sheet-like member, and, it is possible to use a spunbond nonwoven fabric sheet or the like, for example.

The absorbent main body 22 has a function of absorbing excrement such as urine and has a substantially rectangular shape in a plan view. In addition, as shown in FIG. 5, the absorbent main body 22 is arranged at the lateral center while the lengthwise direction is extending along the vertical direction of the diaper 20.

The absorbent main body 22 includes: the liquid-absorbent absorbent core 22a; a core-wrapping sheet (not shown) that covers an outer circumferential surface of the absorbent core 22a; a top sheet 22b; and a back sheet 22c.

The absorbent core 22a is obtained by molding liquid-absorbent fibers (e.g., pulp fibers) into a predetermined shape, and the absorbent core has absorbent polymer (SAP) or the like mixed inside thereof. In addition, as shown in the schematic cross-sectional view of FIG. 5, the absorbent core 22a serves as a portion having a maximum thickness of the diaper 20.

The core-wrapping sheet is a liquid-permeable sheet member that covers the outer surface of the absorbent core 22a, and it is possible to use tissue paper, nonwoven fabric, or the like. Note that the core-wrapping sheet does not have to be necessarily provided.

The top sheet 22b is a liquid-permeable sheet member that is arranged on a skin-side surface of the absorbent core 22a in the thickness direction, and comes into contact with the wearer's skin while the diaper 20 is put on. The top sheet 22b of the present embodiment is formed of an air-through nonwoven fabric sheet, a spunbond nonwoven fabric sheet, or the like.

The back sheet 22c is a liquid-impermeable and moisture-permeable sheet member arranged on the non-skin-side surface of the absorbent core 22a in the thickness direction, and is formed of, for example, a resin sheet. Providing the back sheet 22c suppresses the movement (permeation) of moisture such as urine absorbed by the absorbent main body 22 to the wearer's clothing side.

As shown in FIG. 4, the indicators 23 that can be visually recognized from the non-skin side are provided on the front surface and the back surface of the diaper 20.

The indicator 23 is for visually checking the presence or absence of excrement in a state where the diaper 20 is put on, and examples thereof include a urine indicator for detecting urination, a feces indicator for detecting defecation, and the like.

Generally, the indicator 23 makes the user or the like tell the excrement of urine or feces by a color reaction (change of color) caused by a pH indicator or moisture. That is, the indicator 23 includes a pigment, and when the pigment develops and changes color, the user or the like can tell that urine or feces is excreted.

Generally, when the pigment is exposed to ultraviolet light included in sunlight, internal bonds of the compounds are destroyed and its color fades. That is, when the indicator 23 is exposed to sunlight (ultraviolet light) for a long time, the color reaction of the indicator 23 fades and the color fades, and change in color is reduced, making the user or the like difficult to tell that urine or feces is excreted.

In addition, as shown in FIG. 4, the indicator 23 is positioned in both the front portion 26 and the back portion 27 of the diaper 20, and the vertical length of the indicator 23 may be equal in the front portion 26 and the back portion 27. But in the present embodiment, a front indicator 23a (FIG. 4A) positioned on the front portion 26 side is longer in the vertical direction than a back indicator 23b (FIG. 4B) positioned on the back portion 27 side.

In addition, on the front surface and the back surface of the diaper 20, as shown in FIG. 4, a product graphic 25 that can be visually recognized from the non-skin side is printed. It should be noted that the product graphic 25 is provided in order to make the consumer visually recognize information and the like relating to the diaper 20.

In the present embodiment, the following graphics are provided as the product graphic 25: a first product graphic 25a displaying a name of the diaper 20 (product name: Natural moomy); a second product graphic 25b drawing a flower pattern on the front surface; a third product graphic 25c displaying a feature portion (material used: with organic sheet) of the diaper 20; and a fourth product graphic 25d drawing a flower pattern on the back surface.

In addition, as shown in FIG. 1, a part of the product graphic 25 can be checked from the outside of the containing member 10 through the window region 16. That is, in a state where the container 1 is upright with a lower end of the container 1 being in contact with a ground plane, at least a part of the product graphic 25 can be visually recognized from the outside of the containing member 10. In the present embodiment, the third product graphic 25c and the fourth product graphic 25d can be visually recognized from the outside.

In addition, the product graphic 25 includes a mark such as a manufacturing country of the diaper 20, a logo of a company name or a product name, and a material used. A part of the mark can be checked from the outside. That is, the product graphic 25 includes a mark indicating at least any one of the followings: the name of country in which the diaper 20 has been manufactured: the logo of the company name that manufactures or sells the diaper 20; the logo of the product name of the diaper 20; and the material used. And in a state where the container 1 is upright with the lower end of the container 1 being in contact with a ground plane, at least a part of the mark can be visually recognized from the outside of the containing member 10. In the present embodiment, as the mark, the material used (third product graphic 25c) can be visually recognized from the outside.

In addition, the diaper 20 has a crotch region 28, which is lowest one-fourth of the diaper 20 in the contained state, located on the lower side in the vertical direction (region indicated by downward-right hatched portions in FIG. 4). That is, the diaper 20 is contained so that the crotch side of the diaper 20 is positioned on the lower side of the container 1, and the diaper 20 has the crotch region 28 which is a region located the lowest when the diaper 20 in the contained state is divided into four equal regions in the vertical direction. A skin-side surface of the crotch region 28 is a portion that is in contact with at least the wearer's crotch while the diaper is put on.

### Arrangement of window region 16 and non-window region 17

Next, the following describes a positional relationship (arrangement) between the positions of the window region 16 and the non-window region 17 and other members and the like. Specifically, the following positional relationships in which members overlap or do not overlap with respect to the vertical direction when seen in the front-back direction will be described: the relationship between the upper end and lower end of the window region 16 and the diaper 20 (the center position QL of the diaper 20); the relationship between the non-window region 17 and the indicator 23; and the relationship between the non-window region 17 and the crotch region 28.

Regarding the positional relationship between the upper and lower ends of the window region 16 and the diaper 20 in the vertical direction, the upper end of the window region 16 is positioned below the upper end of the diaper 20, and the lower end of the window region 16 is positioned above the lower end of the diaper 20, as shown in FIG. 1. That is, in a state where the container 1 is upright with the lower end of the container 1 being in contact with a ground plane, the upper end and the lower end of the window region 16 overlap the diaper 20 when seen in the front-back direction.

In addition, the center position QL shown in FIG. 4 indicates the vertical central position of the diaper 20 in the contained state. Regarding the positional relationship between the center position QL and the upper end of the window region 16 in the vertical direction, the center position QL is a position that overlaps the fourth product graphic 25d, and the fourth product graphic 25d is positioned below the upper end of the window region 16, as shown in FIGS. 1 and 4. That is, in a state where the container 1 is upright with a lower end of the container 1 being in contact with a ground plane, the upper end of the window region 16 is positioned above the vertical center position QL of the diaper 20 which is in the contained state.

In the positional relationship between the non-window region 17 and the indicator 23 in the vertical direction, as shown in FIG. 1, a part of the indicator 23 is positioned below the upper end of the non-window region 17 located on the lower side (a dashed line portion of the indicator 23 in FIG. 1). That is, in a state where the container 1 is upright with the lower end of the container 1 being in contact with a ground plane, at least a part of the indicator 23 overlaps the non-window region 17 when seen in the front-back direction.

In addition, a vertical length in which the non-window region 17 and the indicator 23 overlap (vertical length of a portion of the indicator 23 indicated by dashed lines in FIG. 1) is longer than a vertical length in which the window region 16 and the indicator 23 overlap (vertical length of a portion of the indicator 23 indicated by a solid line in FIG. 1). That is, in a state where the container 1 is upright with the lower end of the container 1 being in contact with a ground plane, the vertical length of an overlapping portion where the indicator 23 and the non-window region 17 overlap is longer than the vertical length of an overlapping portion where the indicator 23 and the window region 16 overlap.

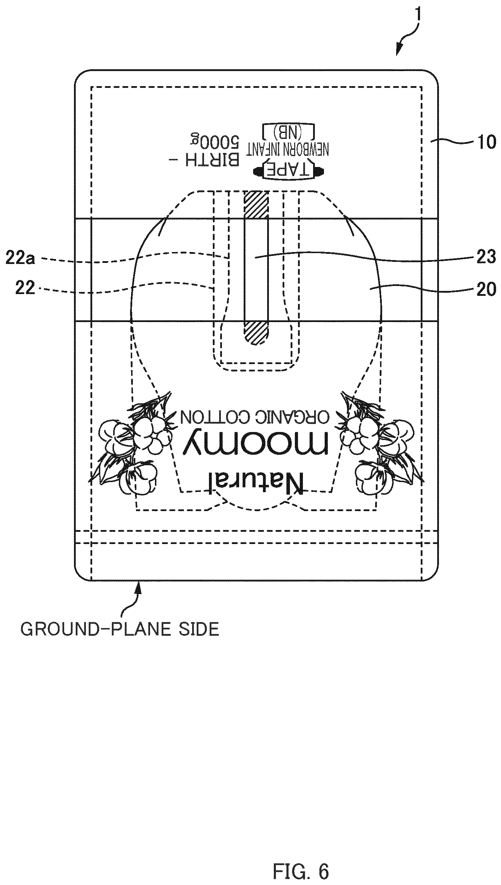

FIG. 6 is a diagram showing a state where the container 1 is reversed in the vertical direction, and is a diagram corresponding to the schematic plan view of FIG. 1. In the containing member 10 according to the present embodiment, the internal space has a gap space, and the diaper 20 can move in the internal space. That is, in the state of FIG. 6, since the diaper 20 moves away from the bottom surface portion 13 and closer to the opening-and-closing seal portion 14 (moves toward the upper side in the container 1), a region of the diaper 20 visually recognizable from the window region 16 varies.

In the positional relationship between the non-window region 17 and the indicator 23 in the vertical direction in the upside-down state, as shown in FIG. 6, a part of the indicator 23 is positioned below the upper end of the non-window region 17 located on the lower side (ground-plane side of FIG. 6), and is positioned above the lower end of the non-window region 17 located on the upper side (opposite side to the ground-plane side of FIG. 6) (regions indicated by upward-right hatched portions in FIG. 6). That is, in a state where the vertical relationship is reversed with respect to the state where the container 1 is upright with the lower end of the container 1 being in contact with a ground plane, at least a part of the indicator 23 overlaps the non-window region 17 when seen in the front-back direction.

Regarding the positional relationship between the non-window region 17 and the crotch region 28 in the vertical direction, as shown in FIGS. 1 and 4, the lower end of the window region 16 is positioned around the lower end of the fourth product graphic 25d, whereas the upper end of the crotch region 28 is positioned below the lower end of the fourth product graphic 25d. That is, in a state where the container 1 is upright with the lower end of the container 1 being in contact with a ground plane, at least a part of the crotch region 28 overlaps the non-window region 17 when seen in the front-back direction.

### Arrangement of graphic 15

Next, the positional relationship (arrangement) between the position of the graphic 15 and other members and the like will be described. Specifically, the following describes the relationship in which each of the absorbent core 22a, the diaper 20, and the gap space overlaps and does not overlap the graphic 15 when seen in the thickness direction of the diaper 20 (direction along the front-back direction, direction penetrating the surface of the paper in the schematic plan view of the left drawing of FIG. 1). In addition, the positional relationship between the opening-and-closing seal portion 14 and the graphic 15 will be described.

It should be noted that, since the diaper 20 according to the present embodiment can move in the internal space of the containing member 10 as mentioned above, the later-described positional relationship between the diaper 20 (absorbent core 22a) and the graphic 15 may be unsatisfied depending on the position in the internal space of the diaper 20. Therefore, the following describes a state in which the diaper 20 is positioned at a certain position among arbitrary positions that can be taken in the internal space (any position in the internal space), and in which that positional relationship is satisfied. It should be noted that, in some cases, the later-described positional relationship between the diaper 20 and the graphic 15 is necessarily satisfied (the positional relationship is always satisfied), and in such a case, the diaper 20 in the internal space may take any position.

In addition, in the following description, the position shown in FIG. 1 will be used as an example of a certain position among any positions that the diaper 20 can take in the internal space. The position of the diaper 20 in the internal space shown in FIG. 1 is a position where the diaper 20 is in contact with the bottom surface portion 13 of the containing member 10 in the vertical direction, and is positioned at the center in the lateral direction.

Regarding the relationship in which the graphic 15 and the absorbent core 22a overlap or does not overlap, for example, as shown in FIG. 1, a part of the fourth graphic 15d overlaps the absorbent core 22a, and the first to third graphics 15a to 15c do not overlap the absorbent core 22a. That is, in a state where the diaper 20 is positioned at a certain position in the containing member 10, at least a part of the graphic 15 does not overlap the absorbent core 22a when seen in the thickness direction of the diaper 20.

Regarding the relationship in which the graphic 15 and the diaper 20 overlap or does not overlap, for example, as shown in FIG. 1, a part of the fourth graphic 15d and the third graphic 15c overlap the diaper 20, and the first graphic 15a and the second graphic 15b do not overlap the diaper 20. That is, in a state where the diaper 20 is positioned at a certain position in the containing member 10, at least a part of the graphic 15 does not overlap the diaper 20 when seen in the thickness direction of the diaper 20.

In addition, for example, as shown in FIG. 1, a part of the fourth graphic 15d and the third graphic 15c overlap the diaper 20, and most parts of the first graphic 15a, the second graphic 15b, and the third graphic 15c do not overlap the diaper 20. That is, in a state where the diaper 20 is positioned at a certain position in the containing member 10, a region where the graphic 15 and the diaper 20 do not overlap when seen in the thickness direction of the diaper 20 is larger than a region where the graphic 15 and the diaper 20 overlap when seen in the thickness direction of the diaper 20.

Regarding the relationship in which the graphic 15 and the gap space overlap or does not overlap, for example, as shown in FIG. 1, the first graphic 15a and the second graphic 15b overlap the gap space. That is, in a state where the diaper 20 is positioned at a certain position in the containing member 10, at least a part of the graphic 15 overlaps the gap space when seen in the thickness direction of the diaper 20.

Regarding the positional relationship between the graphic 15 and the opening-and-closing seal portion 14, the opening-and-closing seal portion 14 does not have the graphic 15. That is, the graphic 15 is not printed on the opening-and-closing seal portion 14.

In addition, the opening-and-closing seal portion 14 is positioned below the upper end of the containing member 10, and the graphic 15 is not provided on the upper side of the opening-and-closing seal portion 14. That is, the graphic 15 is not printed on the upper side of the opening-and-closing seal portion 14.

### Evaluation of container 1

As evaluations of the container 1, the friction coefficient, the surface roughness, and the ink density have been evaluated.

### Friction coefficient evaluation

As for the friction coefficient evaluation, a dynamic friction coefficient MIU was measured and evaluated for the followings: the paper 10a that constitutes the outermost surface of the containing member 10; the resin sheet 10b that constitutes the innermost surface of the containing member 10; the exterior body 21 that constitutes the outermost surface and the facing surface of the diaper 20 (in a state where the diaper 20 is contained, a surface that faces the innermost surface of the containing member 10 is the facing surface. That is, at least a part of the outermost surface of the diaper 20 serves as the facing surface, which faces the containing member 10); and the top sheet 22b that constitutes the located-farthest-on-skin-side surface of the diaper 20. In addition, as for the paper 10a that constitutes the outermost surface of the containing member 10 and the resin sheet 10b that constitutes the innermost surface of the containing member 10, a static friction coefficient µs was also measured. The static friction coefficient µs was measured based on JIS K7125 and was measured using canequim as a counter material.

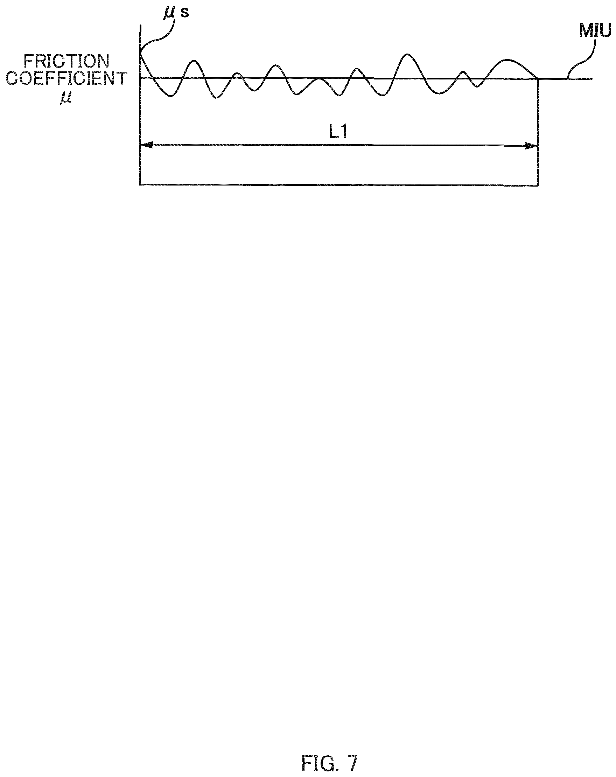

FIG. 7 is a diagram illustrating the friction coefficient µ. A curve shown in FIG. 7 is the friction coefficient µ, and represents a result of measurement with respect to a measurement length L1. Here, the friction coefficient µ can be represented by the following equation with a load W which is applied to a friction block (a portion that comes into contact with a sample) and a frictional force F with the sample. Friction coefficient µ = frictional force F / load W
In addition, the dynamic friction coefficient MIU was defined as the average value (average surface friction coefficient) of the friction coefficient µ in the measurement length L1.

As for a specific evaluation method, five samples were prepared, and the average value of these samples was defined as the dynamic friction coefficient MIU. As a measurement device, there was used a friction-force detecting function of an automated surface tester (KESFB4-AUTO-A manufactured by KATO TECH CO., LTD.). In the measurement conditions, the contact area of the friction block was set to 5 mm × 5 mm (0.25 cm²), and a load W was set to 50 gf (approximately 0.5 N) in the vertical direction including the weight of the friction block. The sample was set in the automated surface tester in a state where a tensile force of 400 g being applied to the sample with respect to its width of 20 cm, and the friction block was brought into contact with the sample with the load W. Then, a frictional force F was measured at a movement rate of the friction block of 1 mm/sec, and the dynamic friction coefficient MIU and the static friction coefficient µs were calculated based on the foregoing equation and the like.

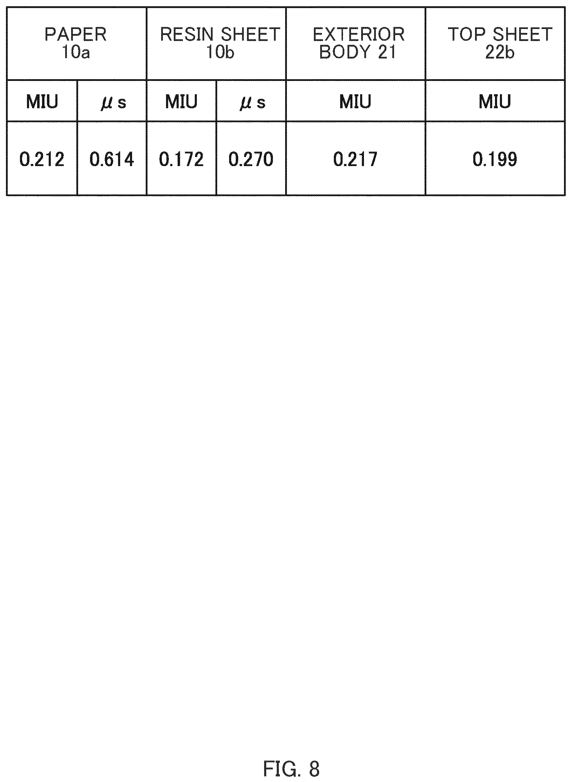

FIG. 8 is a diagram showing measurement results of the friction coefficient. As is shown in this table, the resin sheet 10b has a smaller dynamic friction coefficient MIU than that of the paper 10a. That is, the minimum value of the dynamic friction coefficient MIU on the innermost surface of the containing member 10 (the value of the resin sheet 10b) is smaller than the dynamic friction coefficient MIU on the outermost surface of the containing member 10 formed of the paper 10a.

It should be noted that "the minimum value of the dynamic friction coefficient MIU on the innermost surface of the containing member 10" means the minimum value of the dynamic friction coefficient MIU among multiple regions that constitute the innermost surface if the multiple regions have different materials, surface properties, and the like. For example, in the case where the innermost surface of the containing member is formed of the paper 10a and the resin sheet 10b, the value of the resin sheet 10b, which has a small value of the dynamic friction coefficient MIU, is used.

In addition, the resin sheet 10b has a smaller dynamic friction coefficient MIU than that of the exterior body 21. That is, the minimum value of the dynamic friction coefficient MIU (resin sheet 10b) on the innermost surface of the containing member 10 is smaller than the minimum value of the dynamic friction coefficient MIU (exterior body 21) on the facing surface.

It should be noted that "the minimum value of the dynamic friction coefficient MIU on the facing surface" means the minimum value of the dynamic friction coefficient MIU among multiple regions that constitute the facing surface if the multiple regions have different materials, surface properties, and the like. For example, in the case where a tape whose base material is resin and that is for fixing a rolled diaper when disposing the diaper is provided on the facing surface of the diaper, there is used the value of the tape, which has a small value of the dynamic friction coefficient MIU.

In addition, the paper 10a has a smaller dynamic friction coefficient MIU than that of the exterior body 21. That is, the dynamic friction coefficient MIU on the outermost surface of the paper 10a is smaller than the maximum value of the dynamic friction coefficient MIU (exterior body 21) on the outermost surface of the diaper 20.

It should be noted that "the maximum value of the dynamic friction coefficient MIU on the outermost surface of the absorbent article (diaper 20)" means the maximum value of the dynamic friction coefficient MIU among multiple regions that constitute the outermost surface if the multiple regions have different materials, surface properties, and the like. For example, in the case where a tape whose base material is resin and that is for fixing a rolled diaper when disposing the diaper is provided on the outermost surface of the diaper, the value of the exterior body 21, which has a large value of the dynamic friction coefficient MIU, is used.

In addition, the top sheet 22b has the dynamic friction coefficient MIU smaller than that of the exterior body 21, that is, the minimum value of the dynamic friction coefficient MIU on the located-farthest-on-skin-side surface of the diaper 20 (top sheet 22b) is smaller than the minimum value of the dynamic friction coefficient MIU on the facing surface (exterior body 21).

It should be noted that "the minimum value of the dynamic friction coefficient MIU on the located-farthest-on-skin-side surface of the absorbent article (diaper 20)" means the minimum value of the dynamic friction coefficients MIU among multiple regions that constitute the located-farthest-on-skin-side surface if the multiple regions have different materials, surface properties, and the like. For example, in the case where the located-farthest-on-skin-side surface is constituted by the top sheet 22b and a member different from the top sheet 22b, the value of the member, which has the smallest dynamic friction coefficient MIU, is used.

In addition, the resin sheet 10b has a smaller static friction coefficient µs than that of the paper 10a. That is, the minimum value of the static friction coefficient µs (resin sheet 10b) on the innermost surface of the containing member 10 is smaller than the static friction coefficient µs on the outermost surface of the containing member 10 formed of the paper 10a.

It should be noted that "the minimum value of the static friction coefficient µs on the innermost surface of the containing member 10" means the minimum value of the static friction coefficients µs among multiple regions that constitute the innermost surface if the multiple regions have different materials, surface properties, and the like. For example, in the case where the innermost surface of the containing member is formed of the paper 10a and the resin sheet 10b, the value of the resin sheet 10b, which has a small value of the static friction coefficient µs, is used.

### Surface roughness evaluation

As for the surface roughness evaluation, a surface-roughness average deviation SMD (hereinafter, also simply referred to as a surface roughness SMD) was measured and evaluated for the followings: the paper 10a that constitutes the outermost surface of the containing member 10; the resin sheet 10b that constitutes the innermost surface of the containing member 10; and the exterior body 21 that constitutes the facing surface of the diaper 20.

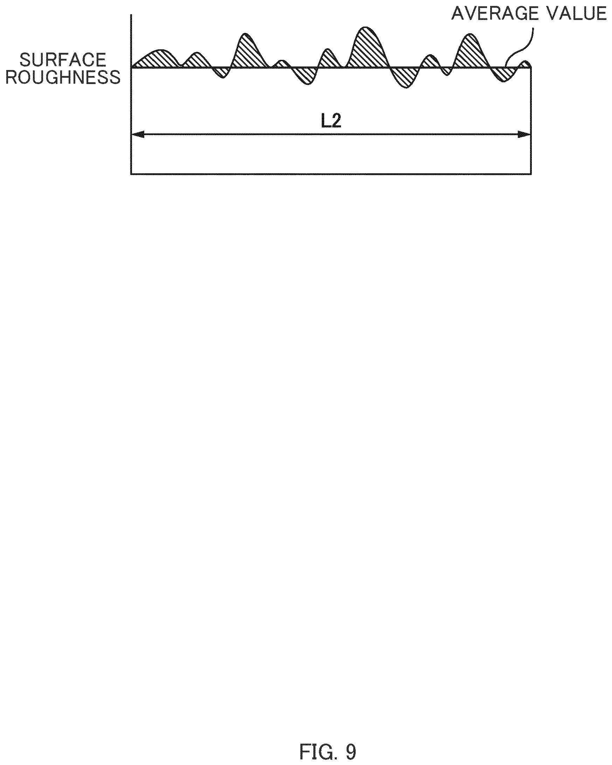

FIG. 9 is a diagram illustrating surface roughness SMD. A curve shown in FIG. 9 is the surface roughness, and represents a result of measurement with respect to a measurement length L2. Here, the surface roughness SMD can be represented by the following equation with an obliquely-upward-right hatched region (a value obtained by integrating a difference from the average value of the surface roughness) shown in FIG. 9 and the measurement length L2. Surface roughness SMD = obliquely-upward-right hatched region / measurement length L2

As a specific evaluation method, five samples were prepared, and average value of these samples was defined as the surface roughness SMD. As a measurement device, there was used a vertical thickness variation detecting function of an automated surface tester (KESFB4-AUTO-A manufactured by KATO TECH CO., LTD.). In the measurement conditions, a wire of 0.5 mm was used as a friction block, a contact surface width was set to 5 mm and a load W was set to 10 gf (approximately 0.1 N) in the vertical direction. The sample was set in the automated surface tester in a state where a tensile force of 400 g being applied to the sample with respect to its width of 20 cm, and the friction block was brought into contact with the sample with the load W. Then, a vertical thickness variation was measured at a movement rate of the friction block of 1 mm/sec, and the surface roughness SMD were calculated based on the foregoing equation and the like.

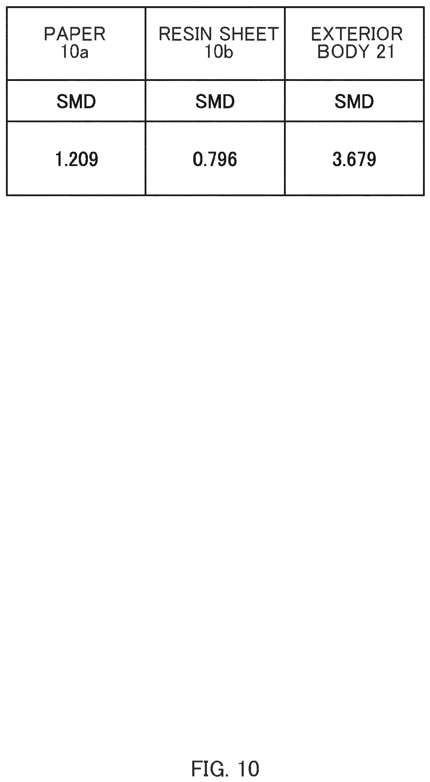

FIG. 10 is a diagram showing measurement results of the surface roughness SMD. As is shown in this table, the resin sheet 10b has a smaller surface roughness SMD than that of the paper 10a. That is, the minimum value of the surface roughness SMD (the value of the resin sheet 10b) on the innermost surface of the containing member 10 is smaller than the surface roughness SMD on the outermost surface of the containing member 10 formed of the paper 10a.

It should be noted that "the minimum value of the surface roughness SMD of the innermost surface of the containing member 10" means the minimum value of the surface roughness SMD among multiple regions that constitute the innermost surface if the multiple regions have different materials, surface properties, and the like. For example, in the case where the innermost surface of the containing member is formed of the paper 10a and the resin sheet 10b, the value of the resin sheet 10b, which has a small value of the surface roughness SMD, is used.

In addition, the resin sheet 10b has a smaller surface roughness SMD than that of the exterior body 21. That is, the minimum value of the surface roughness SMD (resin sheet 10b) on the innermost surface of the containing member 10 is smaller than the minimum value of the surface roughness SMD (exterior body 21) on the facing surface.

It should be noted that "the minimum value of the surface roughness SMD of the facing surface" means the minimum value of the surface roughness SMD among multiple regions that constitute the facing surface if the multiple regions have different materials, surface properties, and the like. For example, in the case where a tape whose base material is a resin and that is for fixing a rolled diaper when disposing the diaper is provided on the facing surface of the diaper, the value of the tape, which has a small value of the surface roughness SMD, is used.

### Ink density evaluation

The ink density evaluation was made by comparing the ink density of the graphic 15 of the containing member 10 (hereinafter, also referred to as the containing-portion graphic 15) and the ink density of the product graphic 25 of the diaper 20. It should be noted that the ink density herein means a darkness of a printed matter on the appearance when the containing-portion graphic 15 or the product graphic 25 are viewed. For example, comparing the case where the product graphic 25 is printed on the outer side of the exterior body 21 of the diaper 20 with the case where the product graphic 25 is printed inside the exterior body 21 in the same manner (with the same darkness), the ink density of the product graphic 25 printed inside is low because the product graphic 25 is seen through the exterior body 21.

Therefore, the ink density of the containing-portion graphic 15 and the product graphic 25 may be compared by a visual recognition method as long as they can be determined based on the appearance darkness. For comparing more strictly, the comparison can be made as follow: for example, images of the containing-portion graphic 15 and the product graphic 25 are captured as a sample by a digital camera under the identical conditions, and this images are monochrome-converted to grayscale images having 256 gradations by image processing software, and the highest value of each image is taken and compared as the maximum ink density.

In addition, in the present embodiment, in terms of appearance, the printed matter of the containing-portion graphic 15 can be more clearly recognized than that of the product graphic 25. That is, the maximum ink density of the containing-portion graphic 15 is higher than the maximum ink density of the product graphic 25.

### First effectiveness of container 1

The container 1 of the diaper 20 according to the present embodiment is a container including: the containing member 10 having the outermost layer, at least a part of the outermost layer being formed of the paper 10a; and the diaper 20 which is contained in the containing member 10 and which has the outermost layer, at least a part of the outermost layer being formed of the nonwoven fabric (exterior body 21), in which the minimum value of the dynamic friction coefficient MIU on the innermost surface of the containing member 10 is smaller than the dynamic friction coefficient MIU on the outermost surface of the containing member 10 formed of the paper 10a. Therefore, this makes it possible to suppress the fluffing of the nonwoven fabric at the time of taking out the diaper 20.

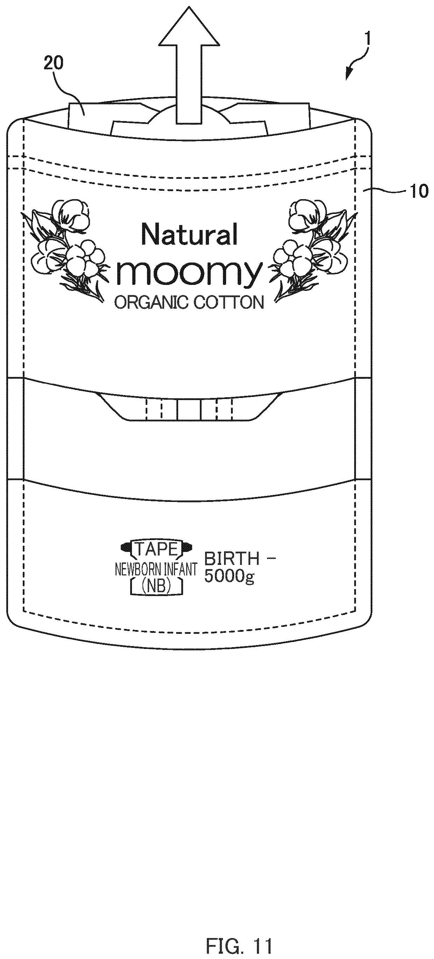

FIG. 11 is a diagram illustrating a state in which the diaper 20 is being taken out from the containing member 10. As shown in FIG. 11, when the diaper 20 is taken out from the containing member 10, the diaper 20 is pulled in a state of opening the opening-and-closing seal portion 14 and spreading the opening portion, taking out the diaper 20 from the containing member 10. That is, the diaper 20 is taken out so as to be pulled out from the containing member 10, and while the facing surface rubbing the innermost surface of the containing member 10.

In addition, a diaper container in which a containing member is formed of paper has been conventionally known. The containing member formed of paper has a high friction coefficient than that of the containing member formed of the resin sheet or the like, and this causes a problem that the diaper is likely to be caught and fluffed, when taking the diaper out from the containing member.

In contrast, in the present embodiment, the minimum value of the dynamic friction coefficient MIU (resin sheet 10b) on the innermost surface of the containing member 10 is smaller than the dynamic friction coefficient on the outermost surface of the containing member 10 formed of the paper 10a.

Compared with the case where the dynamic friction coefficient MIU on the innermost surface is larger than the dynamic friction coefficient MIU on the outermost surface, it is possible to suppress the fluffing of the exterior body 21 at the time of taking out the diaper 20 while maintaining the appearance of the container 1 as paper.

In addition, in the embodiment described above, at least a part of the outermost surface of the diaper 20 is the facing surface that faces the containing member 10, and the minimum value of the dynamic friction coefficient MIU on the innermost surface of the containing member 10 (resin sheet 10b) is smaller than the minimum value of the dynamic friction coefficient MIU on the facing surface (exterior body 21).

Compared with the case where the dynamic friction coefficient MIU on the innermost surface is larger than the dynamic friction coefficient MIU on the facing surface of the diaper 20, this makes it possible to suppress the fluffing of the exterior body 21 at the time of taking out the diaper 20.

In addition, in the embodiment described above, the dynamic friction coefficient MIU on the outermost surface of the paper 10a is smaller than the maximum value of the dynamic friction coefficient on the outermost surface of the diaper 20 (exterior body 21).

Compared with the case where the dynamic friction coefficient MIU on the outermost surface is larger than the dynamic friction coefficient MIU on the facing surface of the diaper 20, it is possible to suppress the fluffing of the exterior body 21 due to the contact of the containing member 10 with the diaper 20 after taking out the diaper 20.

In addition, in the embodiment described above, the minimum value of the surface roughness SMD on the innermost surface of the containing member 10 (the resin sheet 10b) is smaller than the surface roughness SMD on the outermost surface of the containing member 10 formed of the paper 10a.

By doing so, in the innermost surface of the containing member 10, the surface roughness SMD is small, and this suppresses damages on the diaper 20 due to rubbing. In the outermost surface, the surface roughness SMD is large, and this makes the outermost surface more likely to be caught by fingers, making it easier to hold the container 1.

In addition, in the embodiment described above, at least a part of the outermost surface of the diaper 20 is the facing surface that faces the containing member 10, and the minimum value of the surface roughness SMD of the innermost surface of the containing member 10 (resin sheet 10b) is smaller than the minimum value of the surface roughness SMD of the facing surface (exterior body 21).

By doing so, in the innermost surface of the containing member 10, the surface roughness SMD is small, and this suppresses damages on the diaper 20 due to rubbing. In the facing surface of the diaper 20, the surface roughness SMD is large, and this makes the facing surface more likely to be caught by fingers, making it easier to take out the diaper 20 from the containing member 10.

In addition, in the embodiment described above, the upper end portion of the containing member 10 is provided with the opening-and-closing seal portion 14 that is opened when taking out the diaper 20. And in a state where the container 1 is upright with the lower end of the container 1 being in contact with a ground plane, a gap is provided between the diaper 20 and the opening-and-closing seal portion 14.

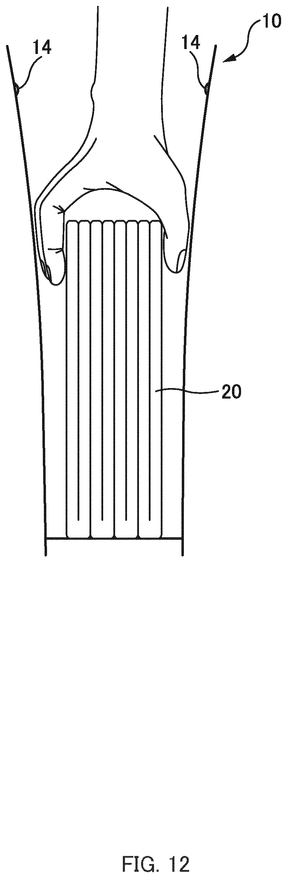

FIG. 12 is a diagram illustrating how the diaper 20 is taken out with putting a hand into a gap of the containing member 10. As shown in FIG. 12, when taking out the diaper 20, it is possible to put a hand into the gap, and to take out the diaper 20 by putting fingers between the containing member 10 and the diaper 20. Accordingly, the containing member 10 is easily spread with fingers, making the diaper 20 less likely to be rubbed when taking it out.

It should be noted that, in a state where the container 1 is upright with the lower end of the container 1 being in contact with a ground plane shown in FIG. 1, the diaper 20 comes into contact with the bottom surface portion 13 of the containing member 10 due to the own weight of the diaper 20, and is positioned at the center in the lateral direction. This state can be achieved, for example, by grabbing and raising the upper end portion of the containing member 10, shaking the upper end portion slightly up and down, and then slowly making the container 1 to be upright.

In addition, in the above description, it has been described that, in the state where the container 1 is upright with a lower end of the container 1 being in contact with a ground plane, the gap is provided between the diaper 20 and the opening-and-closing seal portion 14, but the diaper 20 can be moved in the internal space as described above. That is, the diaper 20 can be brought into contact with the bottom surface portion 13 of the containing member 10 and positioned at an arbitrary position in the internal space in the lateral direction.

In addition, the expression "in the state where the container 1 is upright with the lower end of the container 1 being in contact with a ground plane, the gap is provided between the absorbent article (diaper 20) and the openable portion (the opening-and-closing seal portion 14)" does not mean that, if the diaper 20 moves in the lateral direction inside the containing member 10, "the gap is provided between the absorbent article (diaper 20) and the openable portion (the opening-and-closing seal portion 14)" is satisfied at the entire position. But it means that it is sufficient that the presence of the gap is realized at any one position.

In addition, in the embodiment described above, the opening-and-closing seal portion 14 is positioned below the upper end of the containing member 10.

By doing so, compared with the case where the opening-and-closing seal portion 14 is provided on the upper end of the containing member 10, a further gap is formed (the gap + a portion from the opening-and-closing seal portion 14 to the upper end), and this makes it easier to further spread the containing member 10 with fingers, making the diaper 20 further less likely to be rubbed when taking out.

In addition, in the above embodiment, the container 1 contains a plurality of diapers 20, and the diapers 20 are arranged side by side in the front-back direction, but not being arranged side by side in the vertical direction and the lateral direction.

By doing so, compared with the case where the diapers 20 are arranged in two or more rows, there is no case where one row and another row come into contact with each other, which makes it possible to suppress the collapse of the alignment of the rows of the diapers 20.

In addition, in the embodiment described above, the containing member 10 has a two-layer structure including the paper 10a and the resin sheet 10b, and the innermost surface of the containing member 10 is formed of the resin sheet 10b.

By doing so, the innermost surface becomes a smooth surface, which makes it possible to suppress the fluffing of the exterior body 21 when taking out the diaper 20 from the containing member 10.

In addition, in the embodiment described above, the containing member 10 includes the non-window region 17 and the window region 16, the non-window region 17 being a region in which the paper 10a and the resin sheet 10b overlap, the window region 16 being a region having only the resin sheet 10b, and the window region 16 is provided continuously along the lateral direction from the lateral one end to the lateral other end.

By doing so, the window region 16 having small stiffness is provided continuously from the lateral one end to the lateral other end, and therefore a change in stiffness at a boundary between the window region 16 and the non-window region 17 occurs through from the lateral one end to the lateral other end. This makes such a portion more likely to be fold in the vertical direction, and the folding makes the diaper 20 less likely to move in the containing member 10, making it possible to suppressing the fluffing.

In addition, in the above embodiment, in a state where the container 1 is upright with the lower end of the container 1 being in contact with a ground plane, the upper end and the lower end of the window region 16 overlap the diaper 20 when seen in the front-back direction.

This makes the boundary between the window region 16 and the non-window region 17 more likely to be folded due to a change in stiffness. Accordingly, making the upper end and the lower end overlap the diaper 20 makes such a portion less likely to be folded.

In addition, in the above embodiment, in a state where the container 1 is upright with the lower end of the container 1 being in contact with a ground plane, the upper end of the window region 16 is be positioned above the vertical center of the diaper 20 that is in the contained state.

This makes the boundary between the window region 16 and the non-window region 17 more likely to be folded due to a change in stiffness. This easily makes the containing member 10 compact when disposing, by putting the used diaper 20 into the containing member 10 with the used diaper 20 being folded one time in the vertical direction, for example.

In addition in the above embodiment, the containing member 10 includes: a front side portion 11; a back side portion 12; and a bottom surface portion 13, and a gusset for forming the bottom surface portion 13 is provided between the lower end portion of the front side portion 11 and the lower end portion of the back side portion 12, and the gusset is not provided on the upper end portion of the containing member 10.

That is, since the lower end portion cannot be open (does not have an opening portion), the lower side of the containing member 10 is spread by providing the gusset in advance, which makes it possible to suppress the rubbing of the containing member 10 and the diaper 20 when taking out the diaper 20.

### Second effectiveness of the container 1

The container 1 of the diaper 20 according to the present embodiment is the container 1 having a vertical direction, a lateral direction, and a front-back direction that intersect with each other, the container 1 including: the containing member 10; and the diaper 20 which is contained in the containing member 10 and which includes the indicator 23 for detecting excrement, the containing member 10 including: the window region 16 through which the diaper 20 is capable of being visually recognized from outside; and the non-window region 17 through which the diaper is not capable of being visually recognized, and in a state where the container 1 is upright with the lower end of the container 1 being in contact with a ground plane, at least a part of the indicator 23 overlaps the non-window region 17 when seen in the front-back direction. Accordingly, it is possible to visually recognize the diaper 20 located inside from outside, and to suppress the deterioration of the indicator 23 by suppressing a reaction of the indicator 23 with ultraviolet light at the time of display or the like.

If the diaper is contained in the containing member formed of a non-transparent material (e.g., paper), this causes a problem that it is difficult for the consumer to visually recognize the diaper from the outside when purchasing the diaper.

However, in order to visually recognize the diaper from the outside, the container is configured to have, for example, the transparent window, the diaper is exposed to ultraviolet light of sunlight that is incident from a portion where the diaper can be visually recognized at the time of display or the like. In the case of the diaper which has an indicator for detecting excrement, it causes a problem that the indicator or the like deteriorates due to ultraviolet light of sunlight.

In this regard, in the present embodiment, the window region 16 and the non-window region 17 are provided, and in a state where the container 1 is upright with the lower end of the container 1 being in contact with a ground plane, at least a part of the indicator 23 overlaps the non-window region 17 when seen in the front-back direction.

This makes it possible to visually recognize the diaper 20 located inside from outside. Further, the indicator 23 that overlaps the non-window region 17 at the time of display or the like is suppressed from reacting with ultraviolet light, making it possible to suppress the deterioration of the indicator 23.

It should be noted that, in a state where the container 1 is upright with the lower end of the container 1 being in contact with a ground plane shown in FIG. 1, the diaper 20 comes into contact with the bottom surface portion 13 of the containing member 10 due to the own weight of the diaper 20, and is positioned at the center in the lateral direction. This state can be achieved, for example, by grabbing and raising the upper end portion of the containing member 10, shaking the upper end portion slightly up and down, and then slowly making the container 1 to be upright.

In addition, in the above description, it has been described that, in the state where the container 1 is upright with the lower end of the container 1 being in contact with a ground plane, at least a part of the indicator 23 overlaps the non-window region 17, but the diaper 20 can be moved in the internal space as described above. That is, the diaper 20 can be brought into contact with the bottom surface portion 13 of the containing member 10 and positioned at an arbitrary position in the internal space in the lateral direction.

In addition, the expression "in the state where the container 1 is upright with the lower end of the container 1 being in contact with a ground plane, at least a part of the indicator 23 overlaps the non-window region 17 when seen in the front-back direction" does not mean that, if the diaper 20 moves in the lateral direction inside the containing member 10, "at least a part of the indicator 23 overlaps the non-window region 17 when seen in the front-back direction" is satisfied at the entire position. But it means that it is sufficient that the overlapping is realized at any one position.

In addition, in the above embodiment, the product graphic 25 is printed on the diaper 20, and in a state where the container 1 is upright with the lower end of the container 1 being in contact with a ground plane, at least a part of the product graphic 25 is capable of being visually recognized from the outside of the containing member 10 through the window region 16.

This enables the consumer to check the product graphic 25 of the diaper 20 without opening it at the time of display or the like.

In addition, in the above embodiment, the product graphic 25 include a mark indicating at least any one of followings: a name of country in which the diaper 20 has been manufactured; a logo representing a name of a company that manufactures or sells the diaper 20; a logo representing a product name of the diaper 20; and a material used in the diaper 20.

This enables the consumer to check, without opening it at the time of display or the like, at least one of the followings: the country in which the diaper 20 has been manufactured, the logo of the name of the manufacturing company; the logo of the product name; and the material used.

In addition, in the above embodiment, the diaper 20 includes the front portion 26 that comes into contact with the stomach side of the wearer and the back portion 27 that comes into contact with the back side of the wearer, and the front portion 26 and the back portion 27 face each other, and the diaper 20 is contained so that the crotch side of the diaper 20 is positioned on the lower side of the container 1 and has a crotch region 28 that is a region located lowest when the diaper 20 in the contained state is divided into four pieces in the vertical direction, and in a state where the container 1 is upright with the lower end of the container 1 being in contact with a ground plane, at least a part of the crotch region 28 overlaps the non-window region 17 when seen in the front-back direction.

Since the indicator 23 is particularly required in the crotch region 28, making the crotch region 28 and the non-window region 17 overlap suppresses the reaction of the indicator 23 with ultraviolet light at the time of display or the like. This makes it possible to suppress the deterioration of the indicator 23.

In addition, in the above embodiment, in a state where the container 1 is upright with the lower end of the container 1 being in contact with a ground plane, the vertical length of the overlapping portion where the indicator 23 and the non-window region 17 overlap is longer than the vertical length of the overlapping portion where the indicator 23 and the window region 16 overlap.

Accordingly, compared with the case where the length overlapping with the window region 16 is longer, the reaction of the indicator 23 with the ultraviolet light is suppressed at the time of display, which makes it possible to suppress the deterioration of the indicator 23.

In addition, in the embodiment described above, the containing member 10 has a two-layer structure that is composed of the paper 10a and the resin sheet 10b.

Accordingly, the paper 10a can suppress the deterioration of the indicator 23 caused by ultraviolet light, and the resin sheet 10b can suppress the deterioration of the absorbent core 22a caused by moisture or the like.

In addition, in the embodiment described above, the window region 16 is a region composed of only the resin sheet 10b on which the layer of the paper 10a is not overlaid, and the window region 16 is provided continuously along the lateral direction from the lateral one end to the lateral other end.

Then, since the window region 16 is provided up to the end portion in the width direction, it makes the consumer easier to visually recognize the diaper 20 located inside. In addition, the excellent visibility can reduce the frequency of opening, which makes it possible to reduce the possibility of moisture entering the inside.

In addition, in the above embodiment, in a state where the container 1 is upright with the lower end of the container 1 being in contact with a ground plane, the upper end and the lower end of the window region 16 overlap the diaper 20 when seen in the front-back direction.

This makes the boundary between the window region 16 and the non-window region 17 more likely to be folded due to a change in stiffness. Accordingly, making the upper end and the lower end overlap the diaper 20 makes the boundary less likely to be folded.

In addition, in the above embodiment, in a state where the container 1 is upright with the lower end of the container 1 being in contact with a ground plane, the upper end of the window region 16 is be positioned above the vertical center of the diaper 20 that is in the contained state.

This makes the boundary between the window region 16 and the non-window region 17 more likely to be folded due to a change in stiffness. This easily makes the containing member 10 compact when disposing, by putting the used diaper 20 into the containing member 10 with the used diaper 20 being folded one time in the vertical direction, for example.

In addition, in the above embodiment, in a state where the vertical relationship is reversed from the state where the container 1 is upright with the lower end of the container 1 being in contact with a ground plane, at least a part of the indicator 23 overlaps the non-window region 17 when seen in the front-back direction.

Accordingly, in a state where the container 1 is reversed (since the containing member 10 is formed of paper 10a, there is a gap inside; that is, in a state where the diaper 20 moves upward inside the containing member 10), the reaction of the indicator with the ultraviolet light is suppressed, and this makes it possible to suppress the deterioration of the indicator 23.

In addition, in the embodiment described above, the paper 10a is unbleached.

Accordingly, the paper 10a which is unbleached has a larger content of lignin than bleached paper. Therefore, compared with the case where the bleached paper is used, the reaction of the indicator 23 with ultraviolet light is suppressed, and this makes it possible to suppress the deterioration of the indicator 23.

In addition in the above embodiment, the containing member 10 includes a front side portion 11, a back side portion 12, and a bottom surface portion 13, and a gusset for forming the bottom surface portion 13 is provided between the lower end portion of the front side portion 11 and the lower end portion of the back side portion 12, and the gusset is not provided on the upper end portion of the containing member 10, and the bottom surface portion 13 is joined with at least one of the front side portion 11 and the back side portion 12 in the lower end portion of the containing member 10.

Accordingly, the number of layers where members join in the lower end portion of the containing member 10 is greater than the number of layers where members join in the upper end portion of the containing member 10. Therefore, the lower end portion of the containing member 10 is reinforced, making it possible to suppress the exposure of the indicator 23 due to the damage on the lower end portion and to suppress the deterioration of the indicator 23.

In addition, in the above embodiment, the diaper 20 include the front portion 26 that comes into contact with the stomach side of the wearer and the back portion 27 that comes into contact with the back side of the wearer, and the front portion 26 and the back portion 27 face each other, and the diaper 20 is contained in the containing member 10 so that the crotch side of the diaper 20 is positioned on the lower side of the container 1.

Since the crotch side of the diaper 20 is thick, a portion of the containing member 10 where the crotch side is positioned becomes likely to tear. But, compared with the case where the crotch side is placed on the upper side of the containing member 10, the number of layers where members join in the lower end portion of the containing member 10 is greater than the number of layers where members join in the upper end portion (the lower end portion is more securely joined). This makes it possible to suppress the exposure of the indicator 23 due to the damage on the containing member 10 and to suppress the deterioration of the indicator 23.

### Third effectiveness of the container 1

The container 1 of the diaper 20 according to the present embodiment includes: the containing member 10; and the diaper 20 that is contained in the containing member 10 and that includes the absorbent core 22a, and the graphic 15 is printed on the containing member 10, and in a state where the diaper 20 is positioned at a certain position in the containing member 10, at least a part of the graphic 15 does not overlap the absorbent core 22a when the diaper 20 seen in the thickness direction. Accordingly, when being packed, in which a plurality of containers 1 are arranged, it is possible to suppress the rubbing of the graphic 15 between the adjacent containers 1 or the color transfer of the graphic 15 to the diaper 20.

If a graphic of product information or the like is printed on the containing member, there is a risk, for example, that when a plurality of containers are packed in one cardboard box or the like, it bring into close contact and strongly compress the containers, particularly, a portion of the absorbent core having a maximum thickness of the diaper, and this causes a risk that the graphic is rubbed or ink of the graphic transfers to the diaper.

In contrast, in the above embodiment, in a state where the diaper 20 is positioned at a certain position in the containing member 10, at least a part of the graphic 15 is not overlap the absorbent core 22a when seen in the thickness direction of the diaper 20. That is, by making at least a part of the graphic 15 not overlap with the absorbent core 22a having a maximum thickness in the diaper 20, this makes it possible to suppress the rubbing of the graphic 15 between adjacent containers 1 and the color transfer of the graphic 15 to the diaper 20, when being packed, in which a plurality of containers 1 are arranged side by side.

It should be noted that, the expression "in a state where the absorbent article (diaper 20) is positioned at a certain position in the containing member 10, at least a part of the graphic 15 does not overlap the absorbent core 22a when the absorbent article (diaper 20) seen in the thickness direction" does not mean that, if the diaper 20 moves inside the containing member 10, "at least a part of the graphic 15 does not overlap the absorbent core 22a when the absorbent article (diaper 20) seen in the thickness direction" is satisfied at the entire position. But it means that it is sufficient that the not-overlapping is realized at any one position. The same also applies to the expression "in a state where the absorbent article (diaper 20) is positioned at a certain position in the containing member 10" described later.

In addition, in the above embodiment, in a state where the diaper 20 is positioned at a certain position in the containing member 10, at least a part of the graphic 15 does not overlap the diaper 20 when seen in the thickness direction of the diaper 20.

Accordingly, since the graphic 15 and the diaper 20 do not overlap each other, it is possible to suppress the rubbing of the graphic 15 between the adjacent containers 1 or the color transfer of the graphic 15 to the diaper 20, when being packed, in which a plurality of containers 1 are arranged.

In addition, in the above embodiment, in a state where the diaper 20 is positioned at a certain position in the containing member 10, a region where the graphic 15 and the diaper 20 do not overlap when seen in the thickness direction of the diaper 20 is larger than a region where the graphic 15 and the diaper 20 overlap when seen in the thickness direction of the diaper 20.

Accordingly, compared with the case where the non-overlapping region is small, it is possible to suppress the rubbing of the graphic 15 or the color transfer of the graphic 15 to the diaper 20.

In addition, in the above embodiment, at least a part of the outermost surface of the containing member 10 is formed of paper 10a, and the containing member 10 has an internal space therein, and the internal space has a space where the diaper 20 is present and a gap space where the diaper 20 is not present, and in a state where the diaper 20 is positioned at a certain position in the containing member 10, at least a part of the graphic 15 is overlap the gap space when the diaper 20 is seen in the thickness direction.

Accordingly, since the diaper 20 is not present in the gap space, it is possible to suppress the rubbing of the graphic 15 that overlaps such a portion or the color transfer of the graphic 15 to the diaper 20.

In addition, in the embodiment described above, the containing member 10 has a two-layer structure including the paper 10a and the resin sheet 10b, and the layer of the resin sheet 10b is provided inside the layer of the paper 10a.

Accordingly, the resin sheet 10b makes it possible to suppress the infiltration of ink of the graphic 15 into the internal space, which makes it possible to suppress the color transfer of graphic 15 to the diaper 20.

In addition, in the above embodiment, the minimum value of the static friction coefficient µs on the innermost surface of the containing member 10 is smaller than the static friction coefficient µs on the outermost surface of the containing member 10 formed of the paper 10a.

Accordingly, compared with the case where the minimum value of the static friction coefficient µs on the innermost surface is large, the diaper 20 in the internal space is likely to move inside due to shaking of the container 1 during transport or the like. This makes it possible to suppress the movement of the containing member 10 accompanying with the movement of the diaper 20, which makes it possible to suppress the rubbing of the graphic 15 of the containing member 10.

In addition, in the above embodiment, the container 1 has a vertical direction, a lateral direction, and a front-back direction that intersect with each other, and the containing member 10 includes the front side portion 11, the back side portion 12, and the bottom surface portion 13, and a gusset for forming the bottom surface portion 13 is provided between the lower end portion of the front side portion 11 and the lower end portion of the back side portion 12, and a gusset is not provided on the upper end portion of the containing member 10, and at least a part of the graphic 15 is positioned on the upper portion of the containing member 10.

Thus, the distance between the front side portion 11 and the back side portion 12 at the lateral center is shorter in the upper portion than in the lower portion. Accordingly, printing the graphic 15 on the upper portion makes it possible to widen the space between the graphics 15 of the adjacent containers 1, which makes it possible to suppress the rubbing of the graphic 15 of the containing member 10 or the color transfer of the graphic 15 to the diaper 20.

In addition, in the above embodiment, the container 1 has a vertical direction, a lateral direction, and a front-back direction that intersect with each other, and the upper end portion of the containing member 10 is provided with the opening-and-closing seal portion 14 that is opened when taking out the diaper 20, and the graphic 15 is not printed on the opening-and-closing seal portion 14.

Thus, since the graphic 15 is not printed on the opening-and-closing seal portion 14, which a user comes into contact with when opening the container 1, it is possible to suppress the transfer of ink to the hand and the being faint of the graphic 15.

In addition, in the embodiment described above, the opening-and-closing seal portion 14 is capable of resealing after being opened.

By doing so, the diaper 20 can be put into and taken out by resealing many times. Therefore, by not printing the graphic on the opening-and-closing seal portion 14 that comes into contact many times when putting it in and taking it out, it is possible to further suppress the transfer of ink to the hand and the being faint of the graphic. In addition, even after the container 1 has been opened once, the opening-and-closing seal portion 14 can reseal the containing member 10 each time when it is used, which makes it possible to prevent the entry of moisture. In addition, the being capable of resealing makes it possible to suppress the exposure of the diaper 20 to ultraviolet light from the opening portion.

In addition, in the above embodiment, the opening-and-closing seal portion 14 is positioned below the upper end of the containing member 10, and the graphic 15 is not printed on the upper side of the opening-and-closing seal portion 14.

By doing so, the diaper 20 can be put into and taken out by resealing many times. Therefore, by not printing the graphic 15 on the upper side of the opening-and-closing seal portion 14 that comes into contact many times when putting it in and taking it out, it is possible to further suppress the transfer of ink to the hand and the being faint of the graphic.

In addition, in the above embodiment, the graphic 15 is the containing-portion graphic 15, and the product graphic 25 is printed on the diaper 20, and the maximum ink density of the containing-portion graphic 15 is higher than a maximum ink density of the product graphic 25.

By doing so, compared with the case where the ink density of the containing-portion graphic 15 is low, the containing-portion graphic 15 can be securely displayed, making it easier to visually recognize the graphic (when the ink density is high, the color transfer or the like is more likely to occur, but by the above embodiment in which the containing-portion graphic 15 and the absorbent core 22a do not overlap each other, or the like, it is possible to suppress the being faint of the containing-portion graphic 15 of the containing member 10 or the color transfer of the containing-portion graphic 15 to the diaper 20). In addition, compared with the case where the ink density of the product graphic 25 is high, this makes the consumer to be more likely to have skin-friendly visual impression on the diaper 20.

### Other Embodiments

The above embodiment has been described in order to facilitate the understanding of the present invention, not to interpret the present invention in a limited manner.

In addition, in the embodiment described above, as shown in FIG. 3, the front side portion 11, the back side portion 12, and the bottom surface portion 13 are formed as separate sheets and are joined to each other to form a three-face shape. But the configuration is not limited thereto. For example, a configuration is acceptable in which the front side portion 11 and the bottom surface portion 13 is formed of a single member whose vertical length corresponds to the total vertical length of the front side portion 11 and the bottom surface portion 13, and the bottom surface side of the sheet is folded, forming the bottom surface portion 13.

In addition, in the embodiment described above, the containing member 10 has a three-face shape, but the configuration is not limited thereto. For example, the containing member may have a six-face shape.

In the case of the six-face containing member, unlike the three-face containing member 10, the containing member can usually stand upright with any surface serving as a bottom portion, and accordingly, the concept of the bottom portion may be unclear. That is, in the container including the diaper, the concepts of the vertical direction, the lateral direction, and the front-back direction may be unclear. Therefore, such a six- face containing member is designed so that the consumer can confirm the product information such as the graphic or the like even when any surface is placed as a bottom portion at the time of display or the like. That is, in such a six- face containing member, the working effect of the invention can be obtained, even when any surface becomes the bottom portion.

In addition, in the embodiment described above, the containing member 10 has a two-layer structure of the paper 10a and the resin sheet 10b, but the configuration is not limited thereto. For example, instead of using the resin sheet 10b, the inner surface of the paper 10a may be coated with resin.

In addition, in the embodiment described above, the entirety of the innermost surface of the containing member 10 is a surface made of the resin sheet 10b, but the configuration is not limited thereto. For example, the upper side of the opening-and-closing seal portion 14 may has surfaces made of the paper 10a.

In addition, in the embodiment described above, at least a part of the outermost surface of the diaper 20 is the facing surface that faces the containing member 10, and the minimum value of the dynamic friction coefficient MIU on the located-farthest-on-skin-side surface of the diaper 20 (top sheet 22b) is smaller than the minimum value of the dynamic friction coefficient MIU on the facing surface (exterior body 21). However, the configuration is not limited thereto, and the minimum value of the dynamic friction coefficient MIU on the facing surface may be smaller than the minimum value of the dynamic friction coefficient MIU on the located-farthest-on-skin-side surface of the diaper.

In the former case (case where the value of the top sheet is smaller than the value of the exterior body), compared with the case where the dynamic friction coefficient MIU the farthest on the skin side of the diaper 20 is larger than the dynamic friction coefficient MIU on the facing surface, the wearer is less likely to feel discomfort when the diaper 20 is rubbed while being put on. In addition, when the innermost surface of the containing member and the facing surface of the absorbent article rub, portions of the located-farthest-on-skin-side surface are likely to slide with each other, and accordingly, the facing surface easily moves together with the innermost surface and the fluffing can be suppressed.

In the latter case (case where the value of the exterior body is smaller than the value of the top sheet), compared with the case where the dynamic friction coefficient MIU on the facing surface of the diaper 20 is larger than the dynamic friction coefficient MIU on the located-farthest-on-skin-side surface, the diaper 20 is easily taken out from the containing member 10.

In addition, in the embodiment described above, the diaper 20 is contained so that the back portion 27 of the diaper 20 faces the window region 16, and as shown in FIG. 1, the back portion 27 of the diaper 20 can be visually recognized from the outside of the containing member 10, but the configuration is not limited thereto. The diaper 20 may be contained so that the front portion 26 of the diaper 20 faces the window region 16.

That is, in a state where the container 1 is upright with the lower end of the container 1 being in contact with a ground plane, either one of the front portion 26 and the back portion 27 faces the window region 16. This enables the consumer to check the front surface or the back surface having a larger area than the side surface of the diaper 20 (enables to further visually recognize the entire diaper 20), without opening it at the time of display or the like.

It should be noted that, in a state where the container 1 is upright with the lower end of the container 1 being in contact with a ground plane shown in FIG. 1, the diaper 20 comes into contact with the bottom surface portion 13 of the containing member 10 due to the own weight of the diaper 20, and is positioned at the center in the lateral direction. This state can be achieved, for example, by grabbing and raising the upper end portion of the containing member 10, shaking the upper end portion slightly up and down, and then slowly making the container 1 to be upright.

In addition, in the above description, it has been described that, in a state where the container 1 is upright with the lower end of the container 1 being in contact with a ground plane, either one of the front portion 26 and the back portion 27 faces the window region 16, but the diaper 20 can be moved in the internal space as described above. That is, the diaper 20 can be brought into contact with the bottom surface portion 13 of the containing member 10 and positioned at an arbitrary position in the internal space in the lateral direction.

In addition, the expression "in a state where the container 1 is upright with the lower end of the container 1 being in contact with a ground plane, either one of the front portion 26 and the back portion 27 faces the window region 16" does not mean that, if the diaper 20 moves in the lateral direction inside the containing member 10, "either one of the front portion 26 and the back portion 27 faces the window region 16 when seen in the front-back direction" is satisfied at the entire position. But it means that it is sufficient that the facing the window region 16 is realized at any one position.

In addition, in the above embodiment described above, the diaper 20 is contained so that the back portion 27 of the diaper 20 faces the window region 16, and the front indicator 23a extends upward in the vertical direction longer than the back indicator 23b. But the configuration is not limited thereto. For example, a configuration is acceptable in which the back indicator 23b is longer than the front indicator 23a, and in which the front portion 26 of the diaper 20 and the window region 16 may face each other or the back portion 27 of the diaper 20 and the window region 16 may face each other. That is, the longer portion of the indicator 23 may face the window region 16, or may be on the side opposite to the window region 16 without facing it (the shorter portion face the window region 16).

In other words, that the longer portion of the indicator 23 does not face the window region 16 indicates the following configuration: either one of the front portion 26 and the back portion 27 faces the window region 16, and a portion of the indicator 23 that is positioned on the other one of the front portion 26 and the back portion 27 extends upward in the vertical direction longer than a portion of the indicator 23 that is positioned on the either one of the front portion 26 and the back portion 27. Accordingly, compared with the case where the portion of the indicator 23 having a long length faces the window region 16, it suppresses the reaction of the indicator 23 with ultraviolet light during display or the like (a portion of indicator 23 that overlaps the window region 16 is reduced). This makes it possible to suppress the deterioration of the indicator 23.

In other words, that the longer portion of the indicator 23 faces the window region 16 indicates the following configuration: either one of the front portion 26 and the back portion 27 faces the window region 16, and the portion of the indicator 23 that is positioned on the either one of the front portion 26 and the back portion 27 extends upward in the vertical direction longer than the portion of the indicator 23 that is positioned on the other one of the front portion 26 and the back portion 27. Accordingly, compared with the case where the portion of the indicator 23 having a short length faces the window region 16, it makes the consumer easier to visually recognize the indicator 23 from outside at the time of display or the like, while suppressing the deterioration of the crotch region 28 in the non-window region 17.

In addition, in the case where the longer portion of the indicator 23 faces the window region 16, when the container 1 is upright, the length of the portion where the indicator 23 and the non-window region 17 overlap is longer than the length of the portion where the indicator 23 and the window region 16 overlap. That is, in a state where the container 1 is upright with the lower end of the container 1 being in contact with a ground plane, the vertical length of an overlapping portion where the non-window region 17 overlaps the portion that is positioned on the either one of the front portion 26 and the back portion 27 is longer than the vertical length of an overlapping portion where the window region overlaps the portion that is positioned on the either one of the front portion 26 and the back portion 27. Accordingly, the portion of the indicator 23 that has a long length is capable of being visually recognized from the window region 16, and simultaneously, making longer the length of the region overlapping with the non-window region 17 can suppress the deterioration of the indicator 23, compared with the case of being short.

In addition, in the embodiment described above, the diaper 20 is contained in the containing member 10 so that the crotch side of the diaper 20 is positioned on the lower side of the container 1, but the configuration is not limited thereto. For example, the crotch side of the diaper 20 may be positioned on the upper side. That is, the diaper 20 may be contained in the containing member 10 so that the crotch side of the diaper 20 is positioned on the upper side of the container 1.

In the former case (in the case where the crotch side is on the lower side), the lower side of the containing member 10 is provided with a gusset and can spread, and accordingly, placing the diaper 20 so that the crotch side, which is thick, is located in the lower side, makes it easier to enter the diaper 20.

In the latter case (in the case where the crotch side is the upper side), since the crotch side of the diaper 20 has high stiffness, placing the diaper 20 in the container 1 so that the crotch side is located on the opening-and-closing seal portion 14 side makes it easier to pull the diaper 20 when taking out.

In addition, in the embodiment described above, in a state where the container 1 is upright, the upper end of the window region 16 is below the upper end of the diaper 20, but the configuration is not limited thereto. The upper end of the window region 16 may be above the upper end of the diaper 20. That is, in a state where the container 1 is upright with the lower end of the container 1 being in contact with a ground plane, the upper end of the window region 16 does not have to overlap the diaper 20 when seen in the front-back direction.

This enables the consumer to visually check up to the one end of the diaper 20, without opening it at the time of display of the container 1 or the like.

In addition, in the embodiment described above, as shown in FIG. 2, the graphic 15 is printed on the front side portion 11 of the containing member 10, and the graphic 15 is not printed on the back side portion 12, but the configuration is not limited thereto. The graphic 15 may be printed only on the back side portion 12 without printing the graphic 15 on the front side portion 11. That is, it is sufficient that the graphic 15 is printed on only either one of the front side portion 11 and the back side portion 12. By doing so, compared with the case where the graphic is printed on both of them, it is possible to suppress the rubbing of the graphic 15 of the containing member 10 or the color transfer of the graphic 15 to the diaper 20.

In addition, in the embodiment described above, the opening-and-closing seal portion 14 is capable of resealing, and can be opened and closed a plurality of times, but the configuration is not limited thereto. For example, an openable portion having a perforations and having a structure that cannot be resealed after once opened may be used.

### Other examples

In addition, the following examples are possible.

### (Example 1)

An absorbent-article container including: an absorbent article; and a containing member that contains the absorbent article, wherein the containing member includes an outermost layer and an innermost layer, at least a part of the outermost layer being formed of paper, at least a part of the innermost layer being formed of material having a moisture permeability lower than that of the paper (resin sheet 10b), wherein in the mass of the containing member, the proportion of the mass of the paper that constitutes the outermost layer exceeds 50%.

According to such an absorbent-article container, in addition to the paper, the layer formed of a material having a lower moisture permeability than that of the paper is provided inside, and this makes it possible to suppress moisture from entering the inside of the containing member and to ensure an excellent moisture-proof property. In addition, since the proportion of the mass of the paper exceeds 50%, an identification mark of "paper-made container packaging" is applicable to the container, which makes it possible to provide an environmentally-friendly container.

### (Example 2)

The absorbent-article container has a vertical direction, a lateral direction, and a front-back direction that intersect with each other, wherein the containing member includes a pair of side seal portions and a bottom seal portion, the pair of side seal portions being located in two end portions in the lateral direction and being joined along the vertical direction, the bottom seal portion being located in the lower end portion in the vertical direction and being joined along the lateral direction, wherein in the pair of side seal portions and the bottom seal portion, following low-moisture-permeability materials are welded being adjacent to each other in the front-back direction: the low-moisture-permeability material provided on the innermost layer of one of surfaces that constitute the containing member; and the low-moisture-permeability material provided on the innermost layer of a surface different from the one surface.

According to such an absorbent-article container, in all the seal portions of the containing member, the low-moisture-permeability materials provided on the innermost layers are welded to each other, which makes it less likely for moisture to enter the container.

### (Example 3)

The absorbent-article container has a vertical direction, a lateral direction, and a front-back direction that intersect with each other, wherein the outermost layer is provided above and below the openable portion in the vertical direction, wherein the low-moisture-permeability material provided on the innermost layer is provided above and below the openable portion in the vertical direction.

According to such an absorbent-article container, a portion above the openable portion is a portion to be grabbed when opening the containing member, and, in such an upper portion, the low-moisture-permeability material as well as the outermost layer is provided, making it the grabbed portion less likely to be torn.

### (Example 4)

The absorbent-article container, wherein the containing member includes a window region and a non-window region, the window region being a region at least a part of which is formed of only a resin sheet, the non-window region being a region in which the paper and the resin sheet overlap, wherein the surface area of the window region is smaller than the surface area of the non-window region.

According to such an absorbent-article container, the absorbent article in the container can be confirmed through the window region, and minimizing the window region makes it possible to reduce the possibility of moisture entering the inside of the containing member. In addition, by minimizing the window region, the possibility of being affected by ultraviolet light is reduced.

### (Example 5)

The absorbent-article container, wherein a graphic is provided on the outermost layer, and the graphic is formed of an aqueous ink.

According to the absorbent-article container, using the aqueous ink makes it possible to reduce the amount of a petroleum-derived organic solvent that is used, compared with the case where an image is formed using an oily ink. Accordingly, it is possible to realize a more environmentally friendly container. In addition, since the outermost layer which is a printed medium is formed of paper, the aqueous ink is easily fixed, which makes it possible to stably print even a graphic that looks faint.

### (Example 6)

An absorbent-article container including: an absorbent article; and an containing member that contains the absorbent article, wherein the containing member includes a paper layer, at least a part of which is formed of paper, wherein the at least a part of the paper layer has an unbleached pulp region that is formed of unbleached pulp.

According to the absorbent-article container, the absorbent article is contained in the containing member including the unbleached pulp region, which is difficult to transmit light. This makes it possible to reduce the influence of ultraviolet light on the following likely-turn-yellow constituent members: elastic members such as rubber or urethane that are often included in the absorbent article; a liquid-impermeable film; an ink (ink used in printing on a film, a target tape, an indicator, or the like); and the like. Consequently, it is possible to suppress the yellowing of the absorbent article.

### (Example 7)

The absorbent-article container, wherein a graphic is provided on at least a part of an outer surface of the unbleached pulp region of the containing member.

According to the absorbent-article container, whereas in the case of providing the graphic on the outer surface of the paper layer formed of the bleached pulp, the graphic may be less likely to be seen because the contained absorbent article is seen through, the unbleached pulp region prevents the content from being seen through and makes the graphic easier to be visually recognized because the unbleached pulp region has a large thickness and is in brown or dark brown color.

### (Example 8)

The absorbent-article container, wherein the absorbent-article container has a vertical direction, a lateral direction, and a front-back direction that intersect with each other, wherein a graphic is provided on at least a part of the outermost surface of the absorbent article, and wherein letting the graphic provided in the unbleached pulp region be a containing-member graphic, letting the graphic provided on the absorbent article be a product graphic, in a state where the absorbent article is positioned at a certain position in the containing member, the containing-member graphic and the product graphic have an overlapping portion when seen in the front-back direction.

According to the absorbent-article container, the unbleached pulp region has a large thickness and is in a brown or dark brown color or the like, and therefore, even when the graphic in the unbleached pulp region and the graphic of the absorbent article overlap, the graphic of the absorbent article is not seen through. This makes it possible to maintain visibility of the graphic provided on the unbleached pulp region.

### (Example 9)

An absorbent-article container including: an absorbent article; and an containing member that contains the absorbent article, wherein the containing member includes the outermost layer, at least a part of which is formed of paper, wherein at least a part of a graphic is printed on a region of the outermost layer, the region having a Bekk smoothness less than 20 seconds.

In the above embodiment, a non-coated paper having a region where the Bekk smoothness is less than 20 seconds is used as the outermost layer, and printing is performed so that at least a part of the graphic 15 overlaps the region. It should be noted that the Bekk smoothness is a value expressed by a time (sec) required for a certain amount of atmospheric pressure air to flow between a test piece and a ring-shaped plane in contact with each other in a specific condition, under a specific initial differential pressure. The smaller the Bekk smoothness (the smaller the number of seconds), the lower the smoothness. The Bekk smoothness can be measured based on "Paper and board-Determination of smoothness by Bekk method" specified in JIS P 8119-1998.

According to the standard of JBMS-32-2002 "Cut Sheet Papers for Electrophotographic Copying Machines and Printers" prescribed by the Japan Business Machine and Information System Industries Association, a recommended value of the Bekk smoothness, which serves as a standard when selecting cut paper used for printers and the like, is 20 to 150 seconds. This value does not guarantee the image performance, but is at least a range that serves as a reference when ordinary printing with little being faint is performed. Therefore, in the case where printing is performed on a medium having a smaller Bekk smoothness than 20 seconds, which is the lower limit value of the recommended value, there is a high possibility that being faint will occur in the image.

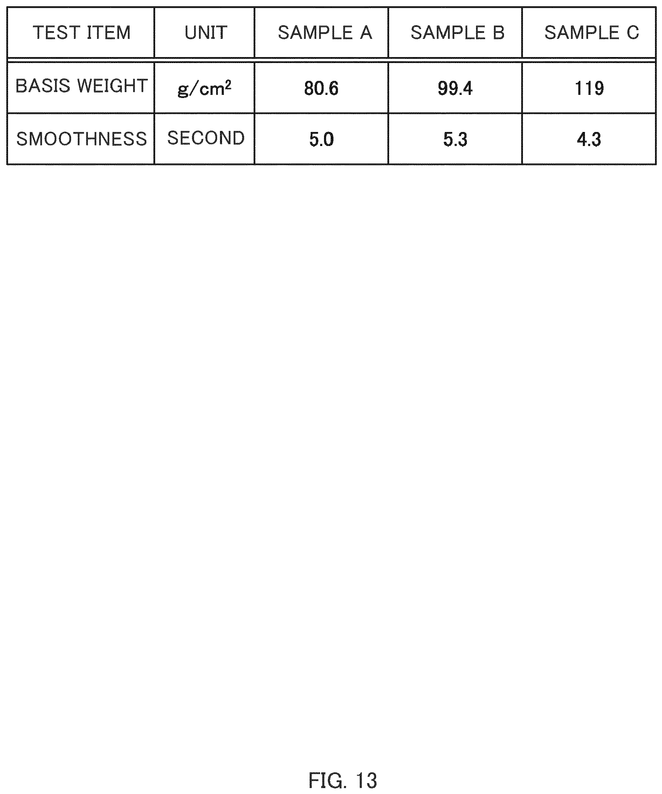

FIG. 13 is a table showing values obtained by measuring the Bekk smoothness of sheet members (samples A to C) of a paper material used as the outermost layer. The Bekk smoothness was measured by the method according to JIS P 8119-1998 described above. As shown in FIG. 13, all of the sheet members A to C have a Bekk smoothness of 5.3 seconds or less, and these are values smaller than 20 seconds, which is the lower limit value of the Bekk smoothness defined in JBMS-32-2002. That is, the smoothness is lower than that of the printing paper recommended in JBMS-32-2002. In addition, it has been confirmed that, when the printing is actually performed using the aqueous ink on these sheet members (Samples A to C), density unevenness is likely to occur in a part of the formed graphic 15, and the graphic 15 appears faint.

Therefore, a sheet member (paper) that has a region having a Bekk smoothness of less than 20 seconds, desirably, 5.3 seconds or less is used as the outermost layer of the containing member 10, and the graphic 15 is printed on that region, and this makes the formed graphic 15 appear faint. Accordingly, this enables the user to be more likely to invoke the texture of material or soft visual impression that the containing member 10 or the absorbent article such as a diaper 20 contained therein is formed of paper.

### (Example 10)

The absorbent-article container, wherein the absorbent-article container has a vertical direction, a lateral direction, and a front-back direction that intersect with each other, wherein the graphic includes a first portion and a second portion that is different from the first portion, wherein the first portion and the second portion have different positions in the front-back direction.

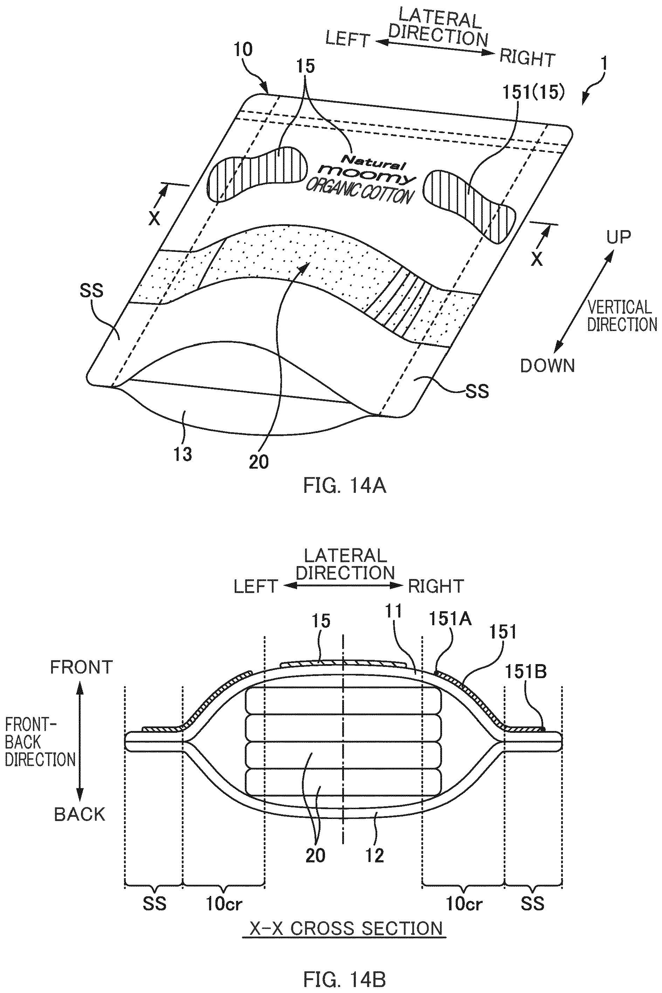

In addition, the adjustment of the arrangement of the graphic 15 printed on the containing member 10 makes it possible to make the user be more likely to invoke soft visual impression of the container 1. FIG. 14A is a perspective view of the container 1 and a diagram showing an arrangement of graphic 15 provided on the containing member 10. FIG. 14B is a schematic crosssectional view showing a cross section taken along a line X-X of FIG. 14A.

As shown in FIG. 1 and 14A, a plurality of pieces of the graphic 15 are provided on the outermost surface of the containing member 10. Among the plurality of pieces of the graphic 15, a graphic provided in a right end portion of FIG. 14A is referred to as a graphic 151. Of the graphic 151, letting a portion positioned on a left end in the lateral direction be a first portion 151A, letting a portion positioned on a right end in the lateral direction be a second portion 151B, the graphic 151 is arranged so that the position of the first portion 151A and the position of the second portion 151B are different in the front-back direction.

The containing member 10 containing the absorbent article has a shape in which a region where the absorbent article is contained expands in the front-back direction. In FIG. 14B, the front side portion 11 and the back side portion 12 are joined to each other in the side seal portions ss in two lateral end portions, which makes the thickness of the containing member 10 in the front-back direction the thinnest. As it goes inward (toward the center side) from two lateral end portions, a space between the front side portion 11 and the back side portion 12 in the front-back direction widens while the front sheet member 11 and the back sheet member 12 are curved. That is, the thickness of the containing member 10 in the front-back direction increases. A region where the front side portion 11 and the back side portion 12 are curved is referred to as a curved region 10cr (see FIG. 14B). In the lateral central part, the thickness of the containing member 10 in the front-back direction is the maximum.

As shown in FIG. 14B, the first portion 151A of the graphic 151 is arranged at a position that overlaps the curved region 10cr, and the second portion 151B is arranged at a position that overlaps the side seal portion ss. As described above, the positions of the first portion 151A and the second portion 151B of the graphic 151 in the front-back direction are different from each other, which makes the graphic 151 to be visually recognized with a stereoscopic feeling in the front-back direction. Therefore, compared with the case where the graphic 151 is visually recognized in a plan view, the shape in which the containing member 10 is three-dimensionally swollen is emphasized, making it more likely to generate soft visual impression of the container 1 as a whole. In particular, since the graphic 151 is arranged so as to widely overlap the curved region 10cr of the containing member 10, the graphic is visually recognized as a rounded curved surface, which makes it possible to give softer impression to the user.

### (Example 11)

The absorbent-article container further comprises a side seal portion, the side seal portion being a portion in which surfaces that constitute the containing member are adjacent in the front-back direction and welded, in two end portions in the lateral direction, the surfaces are different from the each other, wherein the first portion is arranged at a position not overlapping the side seal portion, wherein the second portion is arranged at a position overlapping the side seal portion.

According to the absorbent-article container, the graphic is arranged to straddle a planar region that overlaps the side seal portion and a curved region that does not overlap the side seal portion, which makes the graphic be recognized more complex as a three-dimensional shape. This enables the user to be more likely to invoke soft visual impression of the container.

### (Example 12)

The absorbent-article container, wherein the containing member includes a window portion where the innermost layer is exposed to a front surface side, the window portion being formed by being cut out at least a part of the outermost layer.

According to the absorbent-article container, the user can visually recognize a soft absorbent article such as a paper diaper, through the window portion from the outer side of the container. Accordingly, this enables the user to be more likely to invoke soft visual impression of the container.

### (Example 13)

The absorbent-article container, wherein the innermost layer is overlaid on the back surface side of the outermost layer, the innermost layer being a layer at least a part of which is formed of resin, wherein the average value of dynamic friction coefficient on the innermost layer of the containing member is less than the average value of dynamic friction coefficient on the outermost layer of the containing member.

According to the absorbent-article container, since the average value of the dynamic friction coefficient on the outermost layer of the containing member is large, the surface shape of the outermost layer is likely to be protruded and recessed, which makes it more likely to form (print) a faint graphic. In addition, since the average value of the dynamic friction coefficient on the innermost layer of the containing member is small, a frictional force generated between the innermost layer and the absorbent article is reduced, which makes it possible to smoothly perform an operation of taking out the absorbent article.

### (Example 14)

The absorbent-article container including: the absorbent article; and the containing member that contains the absorbent article, wherein the containing member includes the outermost layer and the innermost layer, at least a part of the outermost layer being formed of paper, at least a part of the innermost layer being formed of resin, wherein the resin that constitutes the innermost layer includes biomass plastic.

According to such an absorbent-article container, the surface (outermost layer) of the containing member is formed of paper, and this enables the user to easily invoke that the container is an environmentally-friendly product. Meanwhile, by forming the back surface (innermost layer) of the containing member with a resin, it is possible to increase the moisture-proof property and to suppress the absorbent article contained therein from getting wet. In addition, since the resin that constitutes the innermost layer composed of biomass plastic, it is possible to reduce the content ratio of a petroleum-derived substance compared with conventional products. Therefore, it is possible to provide the environmentally-friendly absorbent-article container having excellent moisture-proof property.

### (Example 15)

The absorbent-article container, wherein the weight of the biomass plastic included in the resin that constitutes the innermost layer is 10% or more of the weight of the containing member.

According to such an absorbent-article container, it is possible to satisfy the standards for the content ratio of biomass plastic (biomass plastic degree is 10% or more) stipulated in the "biomass plastic identification indicator system" prescribed by the Japan Bioplastic Association. This makes it possible to display a certification mark (biomass mark 10%) of the system, enabling the user to easily recognize that the container is an environmentally-friendly product.

### (Example 16)

The absorbent-article container, wherein at least a part of members that constitute the absorbent article including biomass plastic.

According to such an absorbent-article container, the biomass plastic is included also in the absorbent article, which makes it easier for the user to recognize that it is an environmentally-friendly product and the user can use the absorbent article with peace of mind.

### (Example 17)

The absorbent-article container, wherein the ratio of the weight of biomass plastic included in the containing member to the weight of the containing member is larger than the ratio of the weight of biomass plastic included in the absorbent article to the weight of the absorbent article.

According to such an absorbent-article container, by increasing the content ratio of the biomass plastic in the containing member to be higher than the content ratio of the biomass plastic in the absorbent article, it is possible to reduce the content ratio of the petroleum-derived raw material in the containing member by an amount corresponding thereto. This makes it possible to reduce the amount of the petroleum-derived raw material that is used and to realize the absorbent-article container which is more environmentally friendly.

### (Example 18)

The absorbent-article container has a vertical direction, a lateral direction, and a front-back direction that intersect with each other, wherein in two end portions in the lateral direction and in the lower end portion in the vertical direction of the containing member, following resins are welded being adjacent to each other in the front-back direction: the resin of one of surfaces that constitute the containing member; and the resin provided on the innermost layer of a surface different from the one surface.

According to the absorbent-article container, two side portions in the lateral direction and the lower end portion in the vertical direction of the containing member are firmly sealed by joining the facing resin materials by using a welding means. Accordingly, this makes moisture less likely to enter the inside (containing space) of the container, which makes it possible to enhance moisture-proof property.

### (Example 19)

An absorbent-article container having a vertical direction, a lateral direction, and a front-back direction that intersect with each other, the absorbent-article container including: an absorbent article; and an containing member 110 that contains the absorbent article, wherein the containing member 110 includes an outermost layer and an innermost layer, at least a part of the outermost layer being formed of paper, at least a part of the innermost layer being formed of a resin, wherein the containing member 110 has a gusseted structure in which the containing member 110 is folded inward from two outer sides in the vertical direction so that portions of the outermost layer face each other in the front-back direction, wherein the containing member 110 includes a joining portion and a non-joining portion in two lateral end portions, the joining portion being a portion in which portions of the innermost layer face each other in the front-back direction and are joined, the non-joining portion being a portion in which portions of the outermost layer face each other in the front-back direction and are not joined.

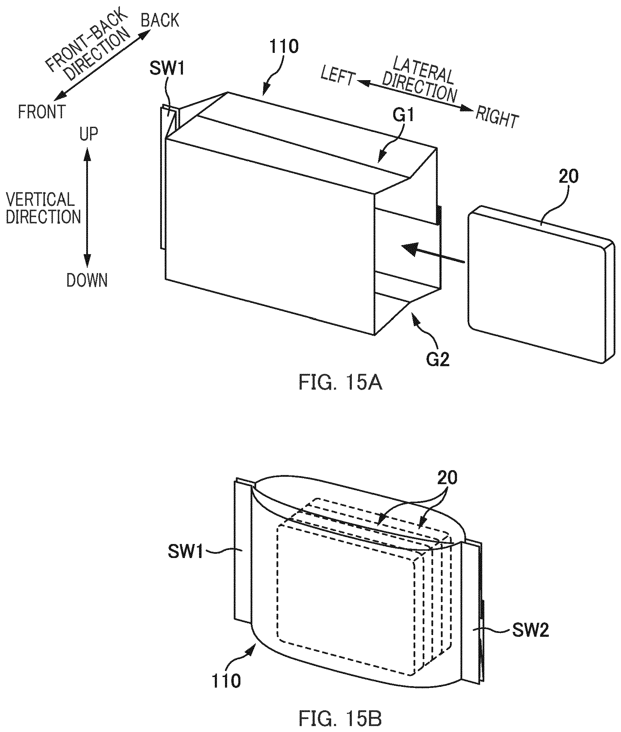

FIG. 15 is a diagram illustrating a containing member 110 that includes gusseted portions G1 and G2. In the containing member 110, as shown in FIG. 15A, on the one end side in the lateral direction, portions of the sheet member that are overlaid in the front-back direction are joined to each other, forming the locking portion SW1. In the locking portion SW1, portions of the innermost layer (resin sheet 10b) which are arranged to face each other in the thickness direction (including the gusseted portions G1 and G2) are welded and joined to each other by using commonly-known welding means such as thermal welding. Both the facing portions of the innermost layer are resin sheet members and have thermoplasticity, and therefore using the welding means such as thermal welding enables to join the facing sheet members to each other with a sufficient strength.

After the end portion on the one side in the lateral direction is locked by the locking portion SW1, the gusseted portions G1 and G2 are unfolded, forming the containing space whose cross section has a substantially rectangular shape. From the other side in the lateral direction, the absorbent article is placed into the containing space and sealing is made by the locking portion SW2. In the containing member 110 having the gusseted structure, a portion of the sheet member that constitutes the exterior where the gusset is folded spreads as a so-called "gusset", which makes it possible to form a wide containing space inside. That is, the volume of the containing space becomes large, which makes it possible to contain a large number of absorbent articles.

In addition, according to the absorbent-article container, in the locking portions provided at two lateral end portions of the containing member 110 that includes paper, the outermost layer has the non-joining portion, and accordingly, its joining strength is not excessively strong compared with the case where the entire locking portion is welded in a conventional, filmmade containing member, making it easier to open the locking portion. In addition, since a package surface is formed of paper, this enables the user to easily invoke that it is an environmentally-friendly product.

### (Example 20)

The absorbent-article container, wherein, in two lateral end portions of the containing member 110, the entirety of a portion in which the portions of the outermost layer face each other in the front-back direction is the non-joining portion.

According to the absorbent-article container, when opening the containing member 110, two facing surfaces (outermost layers) arranged to face each other in the front-back direction are not joined to each other, which makes it easier to separate apart the two facing surfaces in the front-back direction. Accordingly, this enables the user to easily perform an operation of opening the container by peeling off the locking portion.

### (Example 21)

The absorbent-article container, wherein the containing member 110 includes a side seal portion and a back seal portion, the side seal portion being a portion where portions of the resin that constitutes the innermost layer are welded in a state of being adjacent in the front-back direction in two lateral end portions, the back seal portion being a portion where portions of the resin that constitutes the innermost layer are welded in a state of being adjacent in the front-back direction, in a back side portion in the front-back direction.

According to the absorbent-article container, by joining with a welding means portions of resin sheet member having thermoplasticity that face each other, two side portions in the lateral direction and the back side in the front-back direction are firmly sealed. That is, the entirety of the innermost layer that surrounds the containing space of the containing member 110 is sealed. Accordingly, this makes the moisture less likely to enter the inside of the container, making it possible to further enhance moisture-proof property.

### (Example 22)

The absorbent-article container, wherein, in the containing member 110, in a region having a predetermined width from an outermost end in the lateral direction, a portion in which the portions of the innermost layer face each other in the front-back direction are not joined to each other.

According to the absorbent-article container, in the lateral outer end portion, a so-called dry edge is formed, in which portions of the sheet member (innermost layer) are not joined to each other. Accordingly, in the operation of opening the container by peeling off the locking portions that are located at two lateral end portions, the dry edge serves as a point where the peeling starts for peeling off the locking portion (sealed portion), and this makes it possible to perform the opening operation more easily.

### REFERENCE SIGNS LIST

1: container (absorbent-article container),
10: containing member, 11: front side portion, 12: back side portion, 13: bottom surface portion (gusset),
14: opening-and-closing seal portion (openable portion), 15: graphic (containing-portion graphic),
15a: first graphic, 15b: second graphic, 15c: third graphic, 15d: fourth graphic
16: window portion, 17: non-window portion, 20: diaper (absorbent article), 21: exterior body,
22: absorbent main body, 22a: absorbent core, 22b: top sheet, 22c: back sheet
23: indicator, 23a: front indicator, 23b: back indicator,
24: tape, 25: product graphic, 25a: first product graphic, 25b: second product graphic, 25c: third product graphic, 25d: fourth product graphic,
26: front portion, 27: back portion, 28: crotch region, CL: central position, QL: center position,
SS: side seal portion, G1: gusseted portion, G2: gusseted portion 110: containing member

## Claims

1. An absorbent-article container (1) comprising:
a containing member (10) having an outermost surface, at least a part of the outermost surface being formed of paper (10a); and
an absorbent article (20)
that is contained in the containing member (10), and
that has an outermost surface, at least a part of the outermost surface being formed of nonwoven fabric,
**characterized in that** a minimum value of dynamic friction coefficient on an innermost surface of the containing member (10) is less than dynamic friction coefficient on the outermost surface of the containing member (10) formed of the paper.

2. The absorbent-article container (1) according to claim 1, wherein
at least a part of the outermost surface of the absorbent article (20) is a facing surface that faces the containing member (10), and
the minimum value of dynamic friction coefficient on the innermost surface of the containing member (10) is less than a minimum value of dynamic friction coefficient on the facing surface.

3. The absorbent-article container (1) according to claim 1 or 2, wherein
a dynamic friction coefficient on an outermost surface of the paper is less than a maximum value of dynamic friction coefficient on the outermost surface of the absorbent article (20).

4. The absorbent-article container (1) according to any one of claims 1 to 3, wherein
at least a part of the outermost surface of the absorbent article (20) is a facing surface that faces the containing member (10), and
a minimum value of dynamic friction coefficient on a located-farthest-on-skin-side surface of the absorbent article (20) is less than a minimum value of dynamic friction coefficient on the facing surface.

5. The absorbent-article container (1) according to any one of claims 1 to 3, wherein
at least a part of the outermost surface of the absorbent article (20) is a facing surface that faces the containing member (10), and
a minimum value of dynamic friction coefficient on the facing surface is less than a minimum value of dynamic friction coefficient on a located-farthest-on-skin-side surface of the absorbent article (20).

6. The absorbent-article container (1) according to any one of claims 1 to 5, wherein
a minimum value of surface roughness on the innermost surface of the containing member (10) is less than surface roughness on the outermost surface of the containing member (10) formed of the paper.

7. The absorbent-article container (1) according to any one of claims 1 to 6, wherein
at least a part of the outermost surface of the absorbent article (20) is a facing surface that faces the containing member (10), and
a minimum value of surface roughness on the innermost surface of the containing member (10) is less than a minimum value of surface roughness on the facing surface.

8. The absorbent-article container (1) according to any one of claims 1 to 7, wherein
the absorbent-article container (1) has a vertical direction, a lateral direction, and a front-back direction that intersect with each other,
an upper end portion of the containing member (10) is provided with an openable portion (14) that is opened when taking out the absorbent article (20), and
in a state where the container (1) is upright with a lower end of the container (1) being in contact with a ground plane,
a gap is provided between the absorbent article (20) and the openable portion (14).

9. The absorbent-article container (1) according to claim 8, wherein
the openable portion (14) is positioned below an upper end of the containing member (1).

10. The absorbent-article container (1) according to claim 8 or 9, wherein
the container (1) contains a plurality of the absorbent articles (20), and
the absorbent articles are arranged side by side in the front-back direction, but not being arranged side by side in the vertical direction and the lateral direction.

11. The absorbent-article container (1) according to any one of claims 1 to 10, wherein
the containing member (10) has a two-layer structure including the paper and a resin sheet (10b), and
the innermost surface of the containing member (10) is formed of the resin sheet (10b).

12. The absorbent-article container (1) according to claim 11, wherein
the absorbent-article container (1) has a vertical direction, a lateral direction, and a front-back direction that intersect with each other,
the containing member (10) includes a non-window region (17) and a window region (16),
the non-window region (17) being a region in which the paper (10a) and the resin sheet (10b) overlap,
the window region (16) being a region having only the resin sheet (10b), and
the window region (16) is provided continuously along the lateral direction from a lateral one end to a lateral other end.

13. The absorbent-article container (1) according to claim 12, wherein
in a state where the container (1) is upright with a lower end of the container (1) being in contact with a ground plane,
an upper end and a lower end of the window region (16) overlap the absorbent article when seen in the front-back direction.

14. The absorbent-article container (1) according to claim 12, wherein
in a state where the container (1) is upright with a lower end of the container (1) being in contact with a ground plane,
an upper end of the window region (16) is positioned above a vertical center of the absorbent article (20) that is in a contained state.

15. The absorbent-article container (1) according to any one of claims 1 to 14, wherein
the absorbent-article container (1) has a vertical direction, a lateral direction, and a front-back direction that intersect with each other,
the containing member (10) includes a front side portion (11), a back side portion (12), and a bottom surface portion (13),
a gusset for forming the bottom surface portion is provided between a lower end portion of the front side portion (11) and a lower end portion of the back side portion (12), and
the gusset is not provided in an upper end portion of the containing member (10).

16. The absorbent-article container (1) according to claim 15, wherein
the absorbent article (20) includes
a front portion (26) that comes into contact with a stomach side of a wearer, and
a back portion (27) that comes into contact with a back side of the wearer,
the front portion (26) and the back portion (27) face each other, and
the absorbent article (20) is contained in the containing member (10) so that a crotch side of the absorbent article (20) is positioned on a lower side of the container (1).

17. The absorbent-article container (1) according to claim 15, wherein
the absorbent article (20) includes
a front portion (26) that comes into contact with a stomach side of a wearer, and
a back portion (27) that comes into contact with a back side of the wearer,
the front portion (26) and the back portion (27) face each other, and
the absorbent article (20) is contained in the containing member (10) so that a crotch side of the absorbent article (20) is positioned on an upper side of the container (1).

## Patentansprüche

1. Behälter absorbierender Artikel (1), der Folgendes umfasst:
ein aufnehmendes Element (10), das eine äußerste Oberfläche aufweist, wobei zumindest ein Teil der äußersten Oberfläche aus Papier (10a) ausgebildet ist; und
einen absorbierenden Artikel (20),
der in dem aufnehmenden Element (10) enthalten ist und
der eine äußerste Oberfläche aufweist, wobei zumindest ein Teil der äußersten Oberfläche aus Vliesstoff ausgebildet ist,
**dadurch gekennzeichnet, dass** ein minimaler Wert des dynamischen Reibungskoeffizienten auf einer innersten Oberfläche des aufnehmenden Elements (10) geringer ist als der dynamische Reibungskoeffizient auf der äußersten Oberfläche des aufnehmenden Elements (10), die aus dem Papier ausgebildet ist.

2. Behälter absorbierender Artikel (1) nach Anspruch 1, wobei
zumindest ein Teil der äußersten Oberfläche des absorbierenden Artikels (20) eine zugewandte Oberfläche ist, die dem aufnehmenden Element (10) zugewandt ist, und
der minimale Wert des dynamischen Reibungskoeffizienten auf der innersten Oberfläche des aufnehmenden Elements (10) geringer ist als ein minimaler Wert des dynamischen Reibungskoeffizienten auf der zugewandten Oberfläche.

3. Behälter absorbierender Artikel (1) nach Anspruch 1 oder 2, wobei
ein dynamischer Reibungskoeffizient auf der äußersten Oberfläche des Papiers geringer ist als ein maximaler Wert des dynamischen Reibungskoeffizienten auf der äußersten Oberfläche des absorbierenden Artikels (20).

4. Behälter absorbierender Artikel (1) nach einem der Ansprüche 1 bis 3, wobei
zumindest ein Teil der äußersten Oberfläche des absorbierenden Artikels (20) eine zugewandte Oberfläche ist, die dem aufnehmenden Element (10) zugewandt ist, und
ein minimaler Wert des dynamischen Reibungskoeffizienten auf einer Oberfläche, die am weitesten auf der Hautseite vorliegt, des absorbierenden Artikels (20) geringer ist als ein minimaler Wert des dynamischen Reibungskoeffizienten auf der zugewandten Oberfläche.

5. Behälter absorbierender Artikel (1) nach einem der Ansprüche 1 bis 3, wobei
zumindest ein Teil der äußersten Oberfläche des absorbierenden Artikels (20) eine zugewandte Oberfläche ist, die dem aufnehmenden Element (10) zugewandt ist, und
ein minimaler Wert des dynamischen Reibungskoeffizienten auf der zugewandten Oberfläche geringer ist als ein minimaler Wert des dynamischen Reibungskoeffizienten auf einer Oberfläche, die am weitesten auf der Hautseite vorliegt, des absorbierenden Artikels (20).

6. Behälter absorbierender Artikel (1) nach einem der Ansprüche 1 bis 5, wobei
ein minimaler Wert der Oberflächenrauheit auf der innersten Oberfläche des aufnehmenden Elements (10) geringer ist als die Oberflächenrauheit auf der äußersten Oberfläche des aufnehmenden Elements (10), die aus dem Papier ausgebildet ist.

7. Behälter absorbierender Artikel (1) nach einem der Ansprüche 1 bis 6, wobei
zumindest ein Teil der äußersten Oberfläche des absorbierenden Artikels (20) eine zugewandte Oberfläche ist, die dem aufnehmenden Element (10) zugewandt ist, und
ein minimaler Wert der Oberflächenrauheit auf der innersten Oberfläche des aufnehmenden Elements (10) geringer ist als ein minimaler Wert der Oberflächenrauheit auf der zugewandten Oberfläche.

8. Behälter absorbierender Artikel (1) nach einem der Ansprüche 1 bis 7, wobei
der Behälter absorbierender Artikel (1) eine vertikale Richtung, eine laterale Richtung und eine Vorn-Hinten-Richtung aufweist, die einander schneiden,
ein oberer Endteil des aufnehmenden Elements (10) mit einem öffnenden Teil (14) bereitgestellt ist, der beim Herausnehmen des absorbierenden Artikels (20) geöffnet wird, und
in einem Zustand, wo der Behälter (1), mit einem unteren Ende des Behälters (1) im Kontakt mit einer Grundebene, aufrechtsteht,
ein Zwischenraum zwischen dem absorbierenden Artikel (20) und dem öffnenden Teil (14) bereitgestellt ist.

9. Behälter absorbierender Artikel (1) nach Anspruch 8, wobei
der öffnende Teil (14) unterhalb eines oberen Endes des aufnehmenden Elements (1) positioniert ist.

10. Behälter absorbierender Artikel (1) nach Anspruch 8 oder 9, wobei
der Behälter (1) eine Vielzahl der absorbierenden Artikel (20) enthält und
die absorbierenden Artikel in der Vorn-Hinten-Richtung nebeneinander angeordnet sind, doch nicht in der vertikalen Richtung und der lateralen Richtung nebeneinander angeordnet sind.

11. Behälter absorbierender Artikel (1) nach einem der Ansprüche 1 bis 10, wobei
das aufnehmende Element (10) eine zweischichtige Struktur aufweist, die das Papier und eine Harzlage (10b) einschließt, und
die innerste Oberfläche des aufnehmenden Elements (10) aus der Harzlage (10b) ausgebildet ist.

12. Behälter absorbierender Artikel (1) nach Anspruch 11, wobei
der Behälter absorbierender Artikel (1) eine vertikale Richtung, eine laterale Richtung und eine Vorn-Hinten-Richtung aufweist, die einander schneiden,
das aufnehmende Element (10) einen fensterfreien Bereich (17) und einen Fensterbereich (16) einschließt,
wobei der fensterfreie Bereich (17) ein Bereich ist, in dem das Papier (10a) und die Harzlage (10b) überlappen,
wobei der Fensterbereich (16) ein Bereich ist, der nur die Harzlage (10b) aufweist, und
der Fensterbereich (16) durchgehend entlang der lateralen Richtung von einem lateralen Ende zu einem anderen lateralen Ende bereitgestellt ist.

13. Behälter absorbierender Artikel (1) nach Anspruch 12, wobei
in einem Zustand, wo der Behälter (1), mit einem unteren Ende des Behälters (1) im Kontakt mit einer Grundebene, aufrechtsteht,
ein oberes Ende und ein unteres Ende des Fensterbereichs (16) mit dem absorbierenden Artikel bei Ansicht in der Vorn-Hinten-Richtung überlappen.

14. Behälter absorbierender Artikel (1) nach Anspruch 12, wobei
in einem Zustand, wo der Behälter (1), mit einem unteren Ende des Behälters (1) im Kontakt mit einer Grundebene, aufrechtsteht,
ein oberes Ende des Fensterbereichs (16) über einer vertikalen Mitte des absorbierenden Artikels (20), der in einem Aufnahmezustand vorliegt, positioniert ist.

15. Behälter absorbierender Artikel (1) nach einem der Ansprüche 1 bis 14, wobei
der Behälter absorbierender Artikel (1) eine vertikale Richtung, eine laterale Richtung und eine Vorn-Hinten-Richtung aufweist, die einander schneiden,
das aufnehmende Element (10) einen Vorderseitenteil (11), einen Rückseitenteil (12) und einen unteren Oberflächenteil (13) einschließt,
ein Einsatz zur Bildung des unteren Oberflächenteils zwischen einem unteren Endteil des Vorderseitenteils (11) und einem unteren Endteil des Rückseitenteils (12) bereitgestellt ist und
der Einsatz nicht in einem oberen Endteil des aufnehmenden Elements (10) bereitgestellt ist.

16. Behälter absorbierender Artikel (1) nach Anspruch 15, wobei
der absorbierende Artikel (20) Folgendes einschließt:
einen vorderen Teil (26), der mit einer Bauchseite eines Trägers in Kontakt kommt, und
einen hinteren Teil (27), der mit einer Rückenseite des Trägers in Kontakt kommt,
der vordere Teil (26) und der hintere Teil (27) einander zugewandt sind und
der absorbierende Artikel (20) in dem aufnehmenden Element (10) enthalten ist, sodass eine Schrittseite des absorbierenden Artikels (20) auf einer unteren Seite des Behälters (1) positioniert ist.

17. Behälter absorbierender Artikel (1) nach Anspruch 15, wobei
der absorbierende Artikel (20) Folgendes einschließt:
einen vorderen Teil (26), der mit einer Bauchseite eines Trägers in Kontakt kommt, und
einen hinteren Teil (27), der mit einer Rückenseite des Trägers in Kontakt kommt,
der vordere Teil (26) und der hintere Teil (27) einander zugewandt sind und
der absorbierende Artikel (20) in dem aufnehmenden Element (10) enthalten ist, sodass eine Schrittseite des absorbierenden Artikels (20) auf einer oberen Seite des Behälters (1) positioniert ist.

## Revendications

1. Contenant pour article absorbant (1) comportant :
un élément servant à contenir (10) ayant une surface se trouvant le plus à l'extérieur, au moins une partie de la surface se trouvant le plus à l'extérieur étant formée à partir de papier (10a) ; et
un article absorbant (20)
qui est contenu dans l'élément servant à contenir (10), et
qui a une surface se trouvant le plus à l'extérieur, au moins une partie de la surface se trouvant le plus à l'extérieur étant formée à partir d'un tissu non tissé,
**caractérisé en ce qu'**une valeur minimale du coefficient de frottement dynamique sur une surface se trouvant le plus à l'intérieur de l'élément servant à contenir (10) est inférieure au coefficient de frottement dynamique sur la surface se trouvant le plus à l'extérieur de l'élément servant à contenir (10) formé à partir du papier.

2. Contenant pour article absorbant (1) selon la revendication 1, dans lequel
au moins une partie de la surface se trouvant le plus à l'extérieur de l'article absorbant (20) est une surface en regard qui est orientée vers l'élément servant à contenir (10), et
la valeur minimale du coefficient de frottement dynamique sur la surface se trouvant le plus à l'intérieur de l'élément servant à contenir (10) est inférieure à une valeur minimale du coefficient de frottement dynamique sur la surface en regard.

3. Contenant pour article absorbant (1) selon la revendication 1 ou la revendication 2, dans lequel
un coefficient de frottement dynamique sur une surface se trouvant le plus à l'extérieur du papier est inférieur à une valeur maximale du coefficient de frottement dynamique sur la surface se trouvant le plus à l'extérieur de l'article absorbant (20).

4. Contenant pour article absorbant (1) selon l'une quelconque des revendications 1 à 3, dans lequel
au moins une partie de la surface se trouvant le plus à l'extérieur de l'article absorbant (20) est une surface en regard qui est orientée vers l'élément servant à contenir (10), et
une valeur minimale du coefficient de frottement dynamique sur une surface située le plus loin sur le côté orienté vers la peau de l'article absorbant (20) est inférieure à une valeur minimale du coefficient de frottement dynamique sur la surface en regard.

5. Contenant pour article absorbant (1) selon l'une quelconque des revendications 1 à 3, dans lequel
au moins une partie de la surface se trouvant le plus à l'extérieur de l'article absorbant (20) est une surface en regard qui est orientée vers l'élément servant à contenir (10), et
une valeur minimale du coefficient de frottement dynamique sur la surface en regard est inférieure à une valeur minimale du coefficient de frottement dynamique sur une surface située le plus loin sur le côté orienté vers la peau de l'article absorbant (20).

6. Contenant pour article absorbant (1) selon l'une quelconque des revendications 1 à 5, dans lequel
une valeur minimale de la rugosité de surface sur la surface se trouvant le plus à l'intérieur de l'élément servant à contenir (10) est inférieure à la rugosité de surface sur la surface se trouvant le plus à l'extérieur de l'élément servant à contenir (10) formé à partir du papier.

7. Contenant pour article absorbant (1) selon l'une quelconque des revendications 1 à 6, dans lequel
au moins une partie de la surface se trouvant le plus à l'extérieur de l'article absorbant (20) est une surface en regard qui est orientée vers l'élément servant à contenir (10), et
une valeur minimale de la rugosité de surface sur la surface se trouvant le plus à l'intérieur de l'élément servant à contenir (10) est inférieure à une valeur minimale de la rugosité de surface sur la surface en regard.

8. Contenant pour article absorbant (1) selon l'une quelconque des revendications 1 à 7, dans lequel
le contenant pour article absorbant (1) a une direction verticale, une direction latérale et une direction allant d'avant en arrière qui se croisent les unes les autres,
une partie d'extrémité supérieure de l'élément servant à contenir (10) est dotée d'une partie ouvrable (14) qui est ouverte lors du retrait de l'article absorbant (20), et,
dans un état dans lequel le contenant (1) est vertical par rapport à une extrémité inférieure du contenant (1) qui est en contact avec un plan de sol,
un espace est mis en œuvre entre l'article absorbant (20) et la partie ouvrable (14).

9. Contenant pour article absorbant (1) selon la revendication 8, dans lequel
la partie ouvrable (14) est positionnée en dessous d'une extrémité supérieure de l'élément servant à contenir (1).

10. Contenant pour article absorbant (1) selon la revendication 8 ou la revendication 9, dans lequel
le contenant (1) contient une pluralité d'articles absorbants (20), et
les articles absorbants sont agencés côte à côte dans la direction allant d'avant en arrière, mais ne sont pas agencés côte à côte dans la direction verticale et la direction latérale.

11. Contenant pour article absorbant (1) selon l'une quelconque des revendications 1 à 10, dans lequel
l'élément servant à contenir (10) a une structure constituée de deux couches comprenant le papier et une feuille de résine (10b), et
la surface se trouvant le plus à l'intérieur de l'élément servant à contenir (10) est formée à partir de la feuille de résine (10b).

12. Contenant pour article absorbant (1) selon la revendication 11, dans lequel
le contenant pour article absorbant (1) a une direction verticale, une direction latérale et une direction allant d'avant en arrière qui se croisent les unes les autres,
l'élément servant à contenir (10) comprend une région sans fenêtre (17) et une région à fenêtre (16),
la région sans fenêtre (17) étant une région dans laquelle le papier (10a) et la feuille de résine (10b) se chevauchent,
la région à fenêtre (16) étant une région n'ayant que la feuille de résine (10b), et
la région à fenêtre (16) est mise en œuvre en continu le long de la direction latérale depuis une extrémité latérale à une autre extrémité latérale.

13. Contenant pour article absorbant (1) selon la revendication 12, dans lequel,
dans un état dans lequel le contenant (1) est vertical par rapport à une extrémité inférieure du contenant (1) qui est en contact avec un plan de sol,
une extrémité supérieure et une extrémité inférieure de la région à fenêtre (16) chevauchent l'article absorbant lorsque l'on regarde dans la direction allant d'avant en arrière.

14. Contenant pour article absorbant (1) selon la revendication 12, dans lequel,
dans un état dans lequel le contenant (1) est vertical par rapport à une extrémité inférieure du contenant (1) qui est en contact avec un plan de sol,
une extrémité supérieure de la région à fenêtre (16) est positionnée au-dessus d'un centre vertical de l'article absorbant (20) qui est dans un état contenu.

15. Contenant pour article absorbant (1) selon l'une quelconque des revendications 1 à 14, dans lequel
le contenant pour article absorbant (1) a une direction verticale, une direction latérale et une direction allant d'avant en arrière qui se croisent les unes les autres,
l'élément servant à contenir (10) comprend une partie latérale avant (11), une partie latérale arrière (12) et une partie de surface inférieure (13),
un gousset destiné à former la partie de surface inférieure est mis en œuvre entre une partie d'extrémité inférieure de la partie latérale avant (11) et une partie d'extrémité inférieure de la partie latérale arrière (12), et
le gousset n'est pas mis en œuvre dans une partie d'extrémité supérieure de l'élément servant à contenir (10).

16. Contenant pour article absorbant (1) selon la revendication 15, dans lequel
l'article absorbant (20) comprend
une partie avant (26) qui vient se mettre en contact avec le côté ventre d'un utilisateur, et
une partie arrière (27) qui vient se mettre en contact avec un côté dos de l'utilisateur,
la partie avant (26) et la partie arrière (27) sont orientées l'une vers l'autre, et
l'article absorbant (20) est contenu dans l'élément servant à contenir (10) de telle sorte qu'un côté au niveau de l'entrejambe de l'article absorbant (20) est positionné sur un côté inférieur du contenant (1).

17. Contenant pour article absorbant (1) selon la revendication 15, dans lequel
l'article absorbant (20) comprend
une partie avant (26) qui vient se mettre en contact avec le côté ventre d'un utilisateur, et
une partie arrière (27) qui vient se mettre en contact avec un côté dos de l'utilisateur,
la partie avant (26) et la partie arrière (27) sont orientées l'une vers l'autre, et
l'article absorbant (20) est contenu dans l'élément servant à contenir (10) de telle sorte qu'un côté au niveau de l'entrejambe de l'article absorbant (20) est positionné sur un côté supérieur du contenant (1).
